# EUROPÄISCHE PATENTSCHRIFT

(11) **EP 3 223 786 B1**
(45) Veröffentlichungstag und Bekanntmachung des Hinweises auf die Patenterteilung: **18.09.2019**
(21) Anmeldenummer: 15771639.0
(22) Anmeldetag: 02.10.2015
(51) Int. Cl.: A61K 8/49, A61Q 5/06

(54) **MITTEL ZUM FÄRBEN VON KERATINISCHEN FASERN, ENTHALTEND MINDESTENS EINEN DIMEREN, DIKATIONISCHEN AZOFARBSTOFF UND MINDESTENS EIN ANIONISCHES TENSID**
AGENTS FOR DYEING KERATIN FIBRES, CONTAINING AT LEAST ONE DIMERIC, DICATIONIC AZO DYE AND AT LEAST ONE ANIONIC SURFACTANT
AGENTS DE COLORATION DE FIBRES KÉRATINIQUES, CONTENANT AU MOINS UN COLORANT AZOÏQUE DICATIONIQUE DIMÈRE ET AU MOINS UN TENSIOACTIF ANIONIQUE

(30) Priorität: 25.11.2014 DE 102014223939
(43) Veröffentlichungstag der Anmeldung: 04.10.2017
(73) Patentinhaber: Henkel AG & Co. KGaA, 40589 Düsseldorf (DE)
(72) Erfinder: GEBERT-SCHWARZWÄLDER, Antje, 41469 Neuss (DE); GIESA, Helmut, 40670 Meerbusch (DE); KOENEN, Annika, 41516 Grevenbroich (DE)
(86) Internationale Anmeldenummer: PCT/EP2015/072773
(87) Internationale Veröffentlichungsnummer: WO 2016/082998

(56) Entgegenhaltungen:
- WO-A1-2013/068311
- US-A1- 2001 001 333
- US-B2- 7 407 516

## Beschreibung

Die vorliegende Erfindung betrifft Mittel zum Färben von keratinischen Fasern, insbesondere menschlichen Haaren, die (a) mindestens einen dimeren, dikationischen Azofarbstoff einer speziellen Formel (I) in Kombination mit (b) mindestens einem anionischen Tensid enthalten.

Für das Färben von keratinischen Fasern kommen im allgemeinen entweder direktziehende Farbstoffe oder Oxidationsfarbstoffe zur Anwendung. Mit Oxidationsfarbstoffen lassen sich zwar intensive Färbungen mit guten Echtheitseigenschaften erzielen, die Entwicklung der Farbe geschieht jedoch im allgemeinen unter dem Einfluss von Oxidationsmitteln wie z. B. H₂O₂, was in einigen Fällen Schädigungen der Faser zur Folge haben kann. Des Weiteren können einige Oxidationsfarbstoffvorprodukte bzw. bestimmte Mischungen von Oxidationsfarbstoffvorprodukten bei Personen mit empfindlicher Haut sensibilisierend wirken. Direktziehende Farbstoffe werden unter schonenderen Bedingungen appliziert. Ihr Nachteil liegt jedoch darin, dass die Färbungen häufig nur über unzureichende Echtheitseigenschaften verfügen.

Abhängig vom gewünschten Farbergebnis setzt - der Fachmann direktziehende Farbstoffe unterschiedlicher Farbstoffklassen ein. Die aus dem Stand der Technik bekannten direktziehenden Farbstoffe gehören beispielsweise zur Klasse der Nitrofarbstoffe, der Anthrachinonfarbstoffe, der Azofarbstoffe, der Triarylmethanfarbstoffe oder der Methinfarbstoffe. Alle diese Farbstoffklassen sollten für die Anwendung im Kosmetikbereich ein bestimmtes Anforderungsprofil erfüllen. So sollten direktziehende Farbstoffe ein intensives Färbeergebnis liefern und möglichst gute Echtheitseigenschaften besitzen. Durch Umwelteinflüsse sollte das mit direktziehenden Farbstoffen erhaltene Farbergebnis möglichst wenig beeinflusst werden, d.h. die Farbstoffe sollten beispielsweise eine gute Waschechtheit, Lichtechtheit und Reibechtheit besitzen. Auch chemische Einflüsse, welchen die keratinischen Fasern nach dem Färbeprozess ausgesetzt sein können (wie beispielsweise Dauerwellen), sollten das Farbergebnis möglichst wenig verändern.

Um gleichzeitig mit der Färbung auch eine Aufhellung zu erreichen, sollten die direktziehenden Farbstoffe nach Möglichkeit auch mit den bei Blondierverfahren üblicherweise eingesetzten Oxidationsmitteln (wie z.B. Wasserstoffperoxid und/oder Persulfate) kompatibel und gegenüber diesen stabil sein.

Als Farbstoffklasse mit einem herausragenden Anforderungsprofil haben sich kationische Azofarbstoffe herausgestellt. Azofarbstoffe zeichnen sich generell durch eine hohe Stabilität aus. Darüberhinaus besitzen kationische Azofarbstoffe aufgrund ihrer positiven Ladung eine hohe Affinität zur keratinischen Faser, die abhängig von ihrem Schädigungsgrad mehr oder weniger stark negativ geladenen ist.

Sollen keratinische Fasern oxidativ aufgehellt oder blondiert werden, können direktziehende Farbstoffe auch in Kombination mit Oxidationsmitteln eingesetzt werden. Zum Blondieren von Haaren werden in der Regel wässrige Wasserstoffperoxid-Lösungen - entweder allein oder in Kombination mit weiteren, als Bleichaktivatoren wirkenden Oxidationsmitteln wie beispielsweise Persulfatsalzen - auf die Keratinfasern aufgebracht. Um eine ausreichende Blondierwirkung zu erzielen, sind derartige Mittel üblicherweise stark alkalisch eingestellt, der pH-Wert liegt dabei in der Regel zwischen 9 und 10,5. Durch die Einwirkung der Oxidationsmittel werden die Melanine, die natürlichen, farbgebenden Pigmente der Haarfaser, oxidativ zerstört und auf diese Weise eine Entfärbung bzw. Aufhellung der Fasern erreicht. Die Melanine sind im Cortex der Haarfaser lokalisiert und lassen sich in zwei Pigmentklassen einordnen. Eumelanine stellen die erste, bräunlich-schwarzen Pigmentklasse dar, während die rötlichen, schwefelreicheren Pigmente als Pheomelanine bezeichnet werden. Aufgrund der unterschiedlichen Resistenzen der verschiedenen Pigmenttypen gegenüber Oxidationsmitteln werden die Phäo- und Eumelanine jedoch nicht immer gleichmäßig entfärbt. Zudem kann in dunkleren Haaren mit hohem Melaningehalt der Abbau der Melanine nur teilwelse oder unvollständig erfolgen, so dass nach der Blondierung ein Restanteil der farbgebenden Pigmente im Haar verbleibt. In diesen Fällen führt der Restgehalt der nach dem oxidativen Prozess im Haar noch vorhandenen Melanine zu einer gelblichen bis rötlichen Nuancenverschiebung. Daher kommt es insbesondere beim Blondieren von dunkleren Haaren zu einer Farbverschiebung in Richtung warmer (rötlicher) Töne.

Üblicherweise sind solche Farbverschiebungen in Richtung warmer Töne beim Anwender unerwünscht. Daher wird dieser Farbverschiebung zumeist durch eine Tönung in der entsprechenden Komplementärfarbe entgegengewirkt. Ziel ist dabei ein silbrig-kühler Eindruck des Bleichergebnisses. Der Fachmann spricht in diesem Zusammenhang von einer Mattierung.

Zur Mattierung von orange-stichigen Blondnuancen können insbesondere blaue, direktziehende Farbstoffe eingesetzt werden. Für die möglichst vollständige Abschwächung des orangen Farbeindrucks ist es hierbei von Vorteil, wenn der blaue Farbstoff selbst keinen Rotanteil in seiner Färbung besitzt. Farbstoffe in reinen Blautönen sind im Vergleich zu violettstichigen Blaufarbstoffen daher besser zur Mattierung eines zu orangen Blondierergebnisses geeignet.

Innerhalb der Gruppe der in Marktprodukten einsetzbaren direktziehenden Blaufarbstoffe gibt es nur sehr wenige Vertreter, welche die Färbung in reinen Blaunuancen ohne Rotanteil erlauben. Aus dem Stand der Technik sind keine Farbstoffe bekannt, die alle vorgenannten Voraussetzungen in optimaler Weise erfüllen. Es besteht daher weiterhin ein großer Bedarf an stabilen Farbstoffen, welche die keratinischen Fasern in reinen Blautönen färben, und welche ein intensives Farbeergebnis mit heraus ragenden Echtheitseigenschaften liefern.

Aus dem Stand der Technik hinlänglich bekannte monomere kationische Azofarbstoffe sind beispielsweise die Vertreter Basic Orange 31 (Alternativname: 2-[(4-Aminophenyl)azo]-1,3-dimethyl-1H-imidazolium chloride, CAS-Nr, 97404-02-9) und Basic Red 51 (Alternativname: 2-[((4-Dimethylamino)phenyl)azo]-1,3-dimethyl-1H-imidazolium chlorid, CAS-Nr. 77061-58-6).

Beide Farbstoffe färben keratinischen Fasern mit herausragender Farbintensität in orange bis roten Nuancenbereich. Es besteht weiterhin auch noch Bedarf an direktziehenden Blaufarbstoffen, welche mit diesen beiden Farbstoffen in optimaler Weise kompatibel sind.

US 7407516 B2 beschreibt ein Verfahren zum Färben von Keratinfasern, bei welchem ein Mittel enthaltend einen dikationischen Diazofarbstoff einer Formel (I) auf die Keratinfasern aufgetragen wird.

US 2001/0001333 A1 betrifft ein Verfahren zum Färben von Keratinfasern, bei welchem ein Mittel enthaltend einen bestimmten direktziehenden Farbstoff und ein Oxidationsmittel auf den Keratinfasern angewendet wird.

In WO 2013/068311 A1 werden Mittel zum Färben von Haaren mit anionischen Azofarbstoffen thematisiert.

Es war daher die Aufgabe der vorliegenden Erfindung, neuartige Mattierungsfarbstoffe zu finden, welche die üblichen, an direktziehende Farbstoffe gestellten Echtheitsanforderungen erfüllen und welche keratinische Fasern in einem reinen Blauton ohne Rotanteil zu färben vermögen. Die mit diesen Farbstoffen erzielbaren Färbungen sollten eine besonders hohe Farbintensität besitzen.

Darüberhinaus sollen die vorgenannten Blaufarbstoffe auch besonders gut mit den kationischen Azofarbstoffen Basic Orange 31 und Basic Red 51 kompatibel sein.

Überraschenderweise konnte nun gefunden werden, dass diese Aufgabe in hervorragendem Maße erfüllt wird, wenn Farbstoffe der nachfolgend beschriebenen Formel (I) in Kombination mit mindestens einem anionischen Tensid in Mitteln zum Färben von keratinischen Fasern eingesetzt werden.

Ein erster Gegenstand der vorliegenden Erfindung ist ein Mittel zum Färben von keratinischen Fasern, insbesondere menschlichen Haaren, enthaltend in einem kosmetischen Träger
(a) mindestens einen direktziehenden Farbstoff der Formel (I), 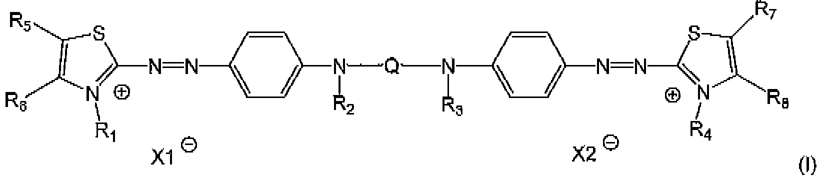 wobei
   - R1, R4: unabhängig voneinander für eine C₁-C₆-Alkylgruppe stehen,
   - R2, R3: unabhängig voneinander für ein Wasserstoffatom oder für eine C₁-C₆-Alkyl-gruppe stehen,
   - R5, R6, R7, R8: Jeweils für ein Wasserstoffatom stehen,
   - X1, X2: unabhängig voneinander für ein physiologisch verträgliches Anion, bevorzugt aus der Gruppe Chlorid, Bromid, Iodid, Methylsulfat, Methylsulfonat, p-Toluolsulfonat, Acetat, Hydrogensulfat, ½ Sulfat oder ½ Tetrachlorozinkat stehen,
   - Q: für eine Gruppierung der Formel (II) steht,

   *-(CH₂)n-* (II)
   - n: für eine ganze Zahl von 3 bis 6 steht,
   und
(b) mindestens ein anionisches Tensid.

Unter keratinischen Fasern, keratinhaltigen Fasern oder Keratinfasern sind Pelze, Wolle, Federn und insbesondere menschliche Haare zu verstehen. Obwohl die erfindungsgemäßen Mittel in erster Linie zum Aufhellen von Keratinfasern geeignet sind, steht prinzipiell einer Verwendung auch auf anderen Gebieten nichts entgegen.

Der erfindungsgemäß verwendete Begriff "Färben von Keratinfasern" umfasst jedwede Form der Farbveränderung der Fasern. Umfasst sind insbesondere die unter den Begriffen Tönung, Blondierung, Mattierung, oxidativer Färbung, semipermanenter Färbung, permanenter Färbung sowie temporärer Färbung umfassten Farbveränderungen. Explizit mit umfasst sind erfindungsgemäß Farbveränderungen, die ein helleres Farbergebnis im Vergleich zur Ausgangsfarbe aufweisen, wie beispielsweise färbende Blondierungen. Unter dem Begriff "Mattierung von Keratinfasern" wird das Entgegenwirken von unerwünschten Nuancenverschiebungen verstanden, welche bei oxidativen Farbveränderungen von Keratinfasern, insbesondere bei Blondierungen oder bei Bleichprozessen, auftreten.

Ziel ist der Mattierung ist die Abschwächung des durch unvollständige Blondierung hervorgerufenen orangen bis rötlichen Farbeindrucks und das Erzeugen einer silbrig-kühlen Farbwahrnehmung nach dem Blondierprozess. Die bei der Mattierung eingesetzten Wirkstoffe können in Form eines Nachbehandlungsschrittes nach dem Blondieren bzw. Bleichen der Keratinfasern appliziert werden. Es ist aber ebenso möglich, die für die Mattierung eingesetzten Wirkstoffe im Rahmen eines einstufigen Verfahrens zusammen mit dem Blondiermittel bzw. dem Bleichmittel auf die Keratinfasern aufzutragen.

Als für die Mattierung geeignete Wirkstoffe können direktziehende Farbstoffe - entweder allein oder im Farbstoffgemisch - mit den geeigneten farblichen Eigenschaften verwendet werden. Darüber hinaus ist es ebenfalls möglich, für die Mattierung direktziehende Farbstoffe in Kombination mit Oxidationsfarbstoffvorprodukten (Entwicklern und Kupplern) einzusetzen.

Die erfindungsgemäßen Mittel enthalten die direktziehenden Farbstoffe der Formel (I) in einem kosmetischen Träger. Dieser kosmetische Träger ist bevorzugt wässrig, alkoholisch oder wässrigalkoholisch. Zum Zwecke der Haarbehandlung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Es ist aber auch möglich, zur Lagerung eine pulverförmige oder auch tablettenförmige Formulierung bereitzustellen. Diese wird dann vor der Anwendung in einem wässrigen Lösungsmittel oder mit organischen Lösungsmitteln bzw. mit Gemischen aus Wasser und organischen Lösungsmitteln unter Erhalt der Anwendungsmischung vermengt. Ein wässriger Träger enthält im Sinne der Erfindung mindestens 40 Gew.-%, insbesondere mindestens 50 Gew.-% Wasser. Unter wässrig-alkoholischen Trägern sind im Sinne der vorliegenden Erfindung wasserhaltige Zusammensetzungen, enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösungsmittel, wie beispielsweise 4-Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel. Bevorzugte erfindungsgemäße Mittel sind dadurch gekennzeichnet, dass sie zusätzlich ein nichtwässriges Lösungsmittel enthalten, wobei bevorzugte erfindungsgemäße Mittel das Lösungsmittel in einer Konzentration von 0,1 bis 30 Gew.-%, bevorzugt in einer Konzentration von 1 bis 20 Gew.-%. ganz besonders bevorzugt in einer Konzentration von 2 bis 10 Gew.-%, jeweils bezogen auf das Mittel, enthalten.

Als ersten wesentlichen Inhaltsstoff (a) enthalten die erfindungsgemäßen Mittel mindestens einen Farbstoff der Formel (I).

Die Substituenten R1 bis R8 der Verbindungen der Formel (I) sind nachstehend beispielhaft erläutert: Beispiele für eine C₁-C₆-Alkylgruppe sind die Gruppen Methyl, Ethyl, Propyl, Isopropyl, n-Butyl, s-Butyl und t-Butyl, n-Pentyl und n-Hexyl. Propyl, Ethyl und Methyl sind bevorzugte Alkylreste.

Die Verbindungen der allgemeinen Formel (I) tragen die Reste R1 und R4 jeweils an dem Stickstoffatom der beiden Thiazolringe. Hierbei können R1 und R4 gleich oder verschieden sein.

Bevorzugt sind die Reste R1 und R4 gleich. R1 und R4 stehen unabhängig voneinander für eine für eine C₁-C₆-Alkylgruppe. Besonders bevorzugt stehen R1 und R4 unabhängig voneinander für eine Methylgruppe oder für eine Ethylgruppe.

Weiterhin tragen die Verbindungen der allgemeinen Formel (I) die Reste R2 und R3; hierbei können R2 und R3 gleich oder verschieden sein. Bevorzugt sind die Reste R2 und R3 gleich.

R2 und R3 stehen unabhängig voneinander für ein Wasserstoffatom oder für eine C₁-C₆-Alkylgruppe. Ganz besonders bevorzugt stehen R2, und R3 unabhängig voneinander für ein Wasserstoffatom, eine Methylgruppe oder eine Ethylgruppe.

Weiterhin tragen die Verbindungen der allgemeinen Formel (I) auch die Reste R5, R6, R7 und R8. Die Reste R5, R6, R7, und R8 stehen alle für ein Wasserstoffatom.

Ein erfindungsgemäßes Mittel ist dadurch gekennzeichnet, dass es (a) mindestens einen direktziehenden Farbstoff der allgemeinen Formel (I) enthält, in der
- R1, R4: unabhängig voneinander für eine C₁-C₆-Alkylgruppe stehen,
- R2, R3: unabhängig voneinander für ein Wasserstoffatom oder für eine C₁-C₆-Alkyl-gruppe stehen,
- R5, R6, R7, R8: jeweils für ein Wasserstoffatom stehen.

In einer ganz besonders bevorzugten Ausführungsform ist ein erfindungsgemäßes Mittel dadurch gekennzeichnet, dass es (a) mindestens einen direktziehenden Farbstoff der allgemeinen Formel (I) enthält, in der
- R1, R4: unabhängig voneinander für eine Methylgruppe oder für eine Ethylgruppe stehen,
- R2, R3: unabhängig voneinander für ein Wasserstoffatom, für eine Methylgruppe oder für eine Ethylgruppe stehen und
- R5, R6, R7, R8: jeweils für ein Wasserstoffatom stehen.

Bei den erfindungsgemäßen Farbstoffen der Formel (I) handelt es sich um dimere Azofarbstoffe, die zweifach positiv geladen sind. Die beiden positiven Ladungen werden durch die anionischen Gegenionen X1 und X2 neutralisiert. Hierbei ist der dikationische organische Teil für die Blaufärbung der Keratinfasern verantwortlich. Die Gegenionen X1 und X2 dienen lediglich der Wahrung der Elektroneutralität, so dass die genaue Natur der Gegenionen X1 und X2 für die Erzielung des gewünschten Farbergebnisses keine wesentliche Rolle spielt. Da der Farbstoff in einem kosmetischen Mittel eingesetzt wird, müssen die Gegenionen X1 und X2 physiologisch verträglich sein. Physiologisch verträglich bedeutet in diesem Zusammenhang zum Einsatz im kosmetischen Mittel (d.h. zur Anwendung auf dem menschlichen Haar und der menschlichen Haut) geeignet. Bei X1 und X2 handelt es sich um physiologisch verträgliche Anionen, bevorzugt aus der Gruppe Chlorid, Bromid, Iodid, Methylsulfat, Methylsulfonat, p-Toluolsulfonat, Acetat, Hydrogensulfat, ½ Sulfat oder ½ Tetrachlorozinkat.

Unter Chlorid wird ein Anion Cl⁻ verstanden. Unter Bromid wird ein Anion Br verstanden. Unter lodid wird ein Anion J⁻ verstanden. Unter Methylsulfat wird ein Anion H₃COSO₄⁻ verstanden. Unter p-Toluolsulfonat wird ein Anion H₃C(C₆H₄)SO₃⁻ verstanden. Unter Acetat wird ein Anion H₃CCOO⁻ verstanden. Unter Hydrogensulfat wird ein AnIon HSO₄⁻ verstanden.

Unter ½ Sulfat wird ein halbes Äquivalent des doppelt negativ geladenen Anions SO₄²⁻ verstanden. Unter ½ Tetrachlorozinkat wird ein halbes Äquivalent des doppelt negativ geladenen Anions Zncl₄²⁻ verstanden. Bei Sulfat und Tetrachlorozinkat ist es demzufolge ebenfalls möglich und auch erfindungsgemäß, wenn der dikationische Farbstoff der Formel (I) durch ein SO₄²⁻ Ion bzw. durch ein ZnCl₄²⁻ Ion neutralisiert wird.

Bei der Gruppierung Q handelt es sich um eine Gruppierung, welche die beiden einfach positiv geladenen Chromophore des Farbstoffs zum dikationischen Dimer verknüpft. Q steht für eine Gruppierung der Formel (II),

*-(CH₂)n-* (II)

- n: für eine ganze Zahl von 3 bis 6 steht.

Die beiden mit einem Stern markierten Position stellen hierbei jeweils die Verknüpfungspositionen zu den beiden N-Atomen der Formel (I) dar.

Überraschenderweise hat es sich für die Erzielung eines intensiven Farbergebnisses als grundsätzlich wichtig und erfindungswesentlich herausgestellt, dass die verbindende Gruppierung Q, welche die beiden einzelnen Chromophore miteinander verknüpft, eine Kettenlänge von mindestens 3 Atomen aufweist.

Aus diesem Grund steht n in der Formel (II) für eine ganze Zahl von mindestens 3. Die verbindende Gruppierung Q der Formel (II) umfasst demnach mindestens 3 C-Atome (d.h. hierbei handelt es sich um eine Gruppierung mit der Mindestlänge von -CH₂-CH₂-CH₂-).

Im Rahmen von Vergleichsversuchen hat sich herausgestellt, dass dimere Azofarbstoffe des prinzipiellen Typs der Formel (I), die jedoch eine nicht erfindungsgemäße Linker-Gruppe Q mit einer Länge von nur 2 C-Atomen besitzen, ein extrem schlechtes Farbaufzugsvermögen auf die Keratinfasern besitzen.

Während mit den erfindungsgemäßen Farbstoffen der Formel (I) intensive Färbungen mit tief dunkelblauem Farbton erzielt werden konnten, führten Färbungen mit analogen dimeren Farbstoffen, die über eine kürzere Gruppierung Q mit einer Kettenlänge von nur 2 C-Atomen verknüpft sind, zu praktisch überhaupt keinem Farbaufzug auf den keratinischen Fasern.

Ohne auf eine Theorie beschränkt zu sein, könnte möglicherweise eine mit der kurzen Linker-Kette Q verbundene starre Geometrie und hierdurch bedingt eine ungünstige räumliche Konformation des Farbstoffes die Diffusion der kurzkettigen dimeren Farbstoffe in die keratinische Faser hinein verschlechtern.

Ein erfindungsgemäßes Mittel ist dadurch gekennzeichnet, dass es (a) mindestens einen direktziehenden Farbstoff der allgemeinen Formel (I) enthält, in der Q für eine Gruppierung der Formel (II) steht,

*-(CH₂)n-* (II)

und
- n: jeweils unabhängig voneinander für eine ganze Zahl von 3 bis 6 steht.

In einer explizit ganz besonders bevorzugten Ausführungsform ist ein erfindungsgemäßes Mittel dadurch gekennzeichnet, dass es (a) mindestens einen Farbstoff der allgemeinen Formel (I) enthält, in der
- Q: für eine Gruppierung der Formel (II) steht,

*-(CH₂)n-* (II)
und
- n: für die Zahl 3 steht.

In einer weiteren bevorzugten Ausführungsform ist ein Mittel zum Färben von keratinischen Fasern dadurch gekennzeichnet, dass es mindestens eine Verbindung der allgemeinen Formel (I) enthält, die ausgewählt ist aus
- Salzen des 3-Methyl-2-[2-(4-{[3-({4-[2-(3-methyl-1,3-thiazol-3-ium-2-yl)diazen-1-yl]phenyl}amino)propyl]amino}phenyl)diazen-1-yl]-1,3-thiazol-3-iums 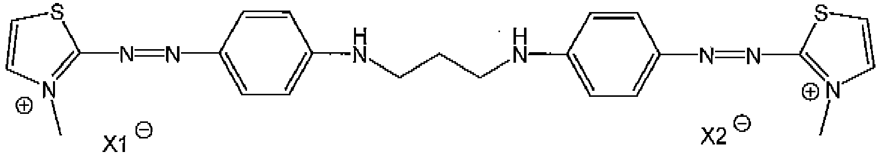
- Salzen des 3-Methyl-2-[2-(4-{[4-({4-[2-(3-methyl-1,3-thiazol-3-ium-2-yl)diazen-1-yl]phenyl}amino)butyl]amino}phenyl)diazen-1-yl]-1,3-thiazol-3-iums 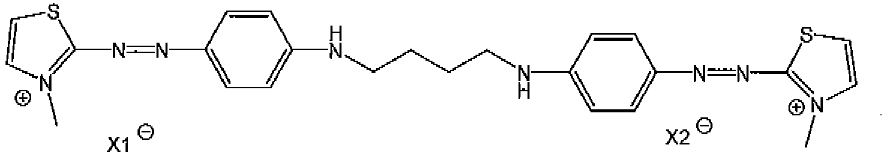
- Salzen des 3-Methyl-2-[2-(4-{[5-({4-[2-(3-methyl-1,3-thiazol-3-ium-2-yl)diazen-1-yl]phenyl}amino)pentyl]amino}phenyl)diazen-1-yl]-1,3-thiazol-3-iums 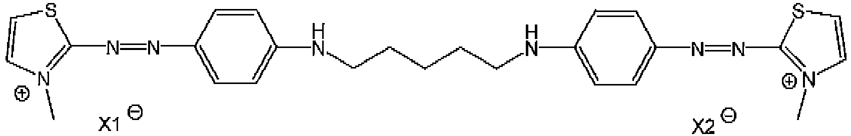
- Salzen des 3-Methyl-2-(2-{4-[methyl({3-[methyl({4-[2-(3-methyl-1,3-thiazol-3-ium-2-yl)diazen-1-yl]phenyl})amino]propyl})amino]phenyl}diazen-1-yl)-1,3-thiazol-3-iums 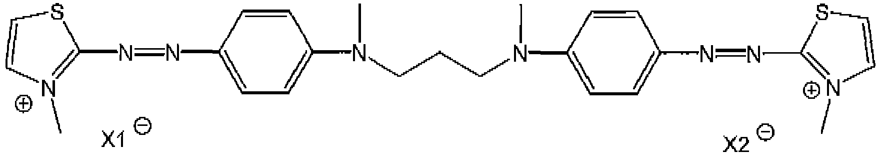
- Salzen des 3-Methyl-2-(2-{4-[methyl({4-[methyl({4-[2-(3-methyl-1,3-thiazol-3-ium-2-yl)diazen-1-yl]phenyl})amino]butyl})amino]phenyl}diazen-1-yl)-1,3-thiazol-3-iums 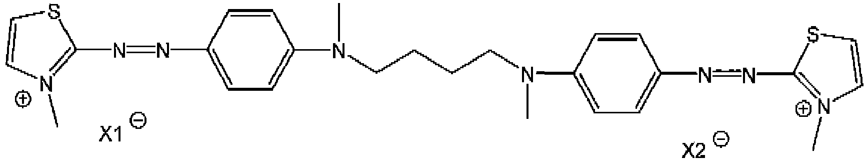
- Salzen des 3-Methyl-2-(2-{4-[methyl({5-[methyl({4-[2-(3-methyl-1,3-thiazol-3-ium-2-yl)diazen-1-yl]phenyl})amino]pentyl})amino]phenyl}diazen-1-yl)-1,3-thiazol-3-iums 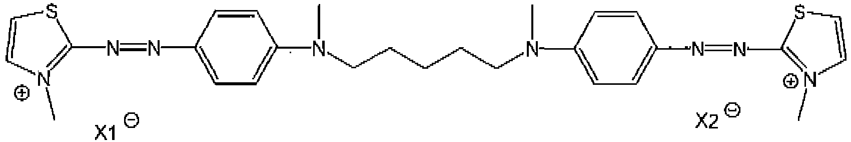
- Salzen des 2-(2-{4-[Ethyl({3-[ethyl({4-[2-(3-methyl-1,3-thiazol-3-ium-2-yl)diazen-1-yl]phenyl})amino]propyl})amino]phenyl}diazen-1-yl)-3-methyl-1,3-thiazol-3-iums 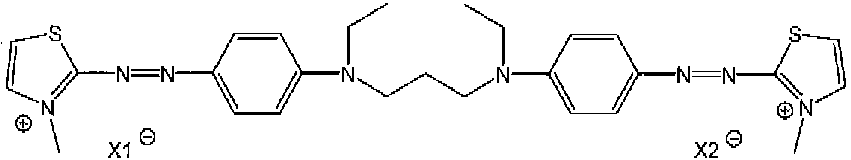
- Salzen des 2-(2-(4-[Ethyl({4-[ethyl({4-[2-(3-methyl-1,3-thiazol-3-ium-2-yl)diazen-1-yl]phenyl})amino]butyl})amino]phenyl}diazen-1-yl)-3-methyl-1,3-thiazol-3-iums 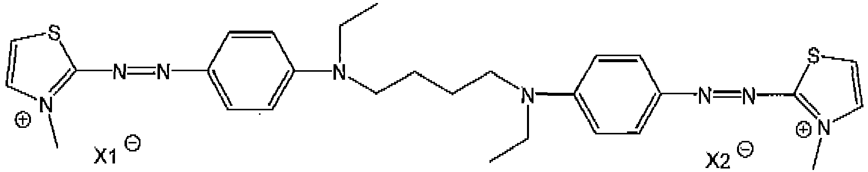
- Salzen des 2-(2-{4-[Ethyl({5-[ethyl({4-[2-(3-methyl-1,3-thiazol-3-ium-2-yl)diazen-1-yl]phenyl})amino]pentyl})amino]phenyl}diazen-1-yl)-3-methyl-1,3-thiazol-3-iums 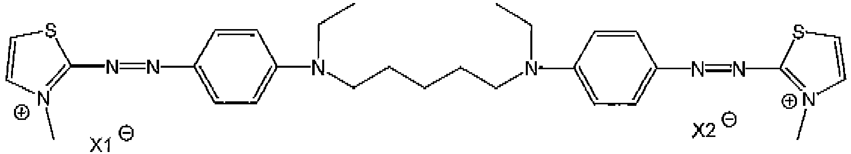
- Salzen des 3-Ethyl-2-[2-(4-{[3-({4-[2-(3-ethyl-1,3-thiazol-3-ium-2-yl)diazen-1-yl]phenyl}amino)propyl]amino}phenyl)diazen-1-yl]-1,3-thiazol-3-iums 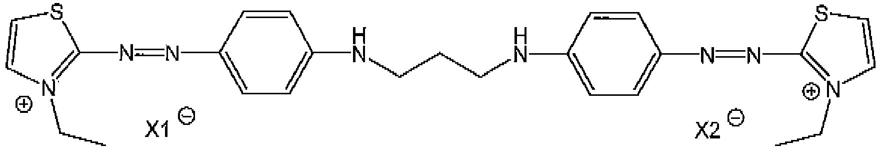
- Salzen des 3-Ethyl-2-[2-(4-{[4-({4-[2-(3-ethyl-1,3-thiazol-3-ium-2-yl)diazen-1-yl]phenyl}amino)butyl]amino}phenyl)diazen-1-yl]-1,3-thiazol-3-iums 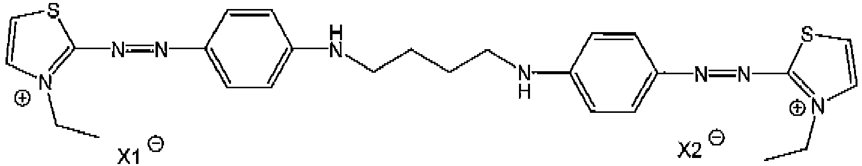
- Salzen des 3-Ethyl-2-[2-(4-{[5-({4-[2-(3-ethyl-1,3-thiazol-3-ium-2-yl)diazen-1-yl]phenyl}amino)pentyl]amino}phenyl)diazen-1-yl]-1,3-thiazol-3-iums 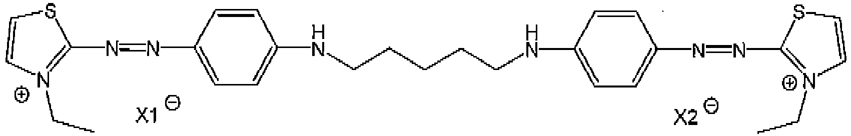
- Salzen des 3-Ethyl-2-[2-(4-{[3-({4-[2-(3-ethyl-1,3-thiazol-3-ium-2-yl)diazen-1-yl]phenyl}(methyl)amino)propyl](methyl)amino}phenyl)diazen-1-yl]-1,3-thiazol-3-iums 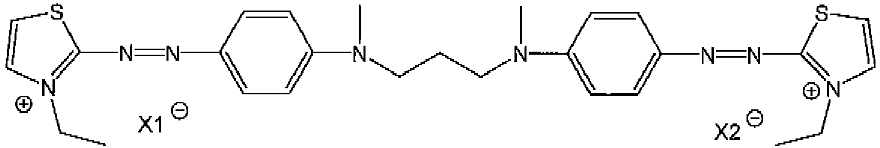
- Salzen des 3-ethyl-2-[2-(4-([4-({4-[2-(3-ethyl-1,3-thiazol-3-ium-2-yl)diazen-1-yl]phenyl}(methyl)amino)butyl](methyl)amino}phenyl)diazen-1-yl]-1,3-thiazol-3-iums 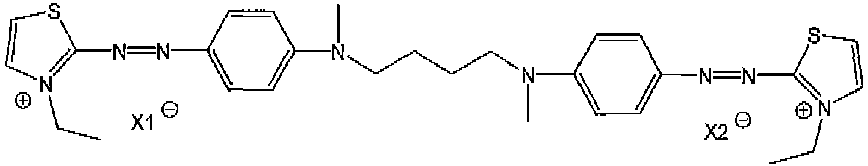
- Salzen des 3-Ethyl-2-[2-(4-([5-({4-[2-(3-ethyl-1,3-thiazol-3-lum-2-yl)diazen-1-yl]phenyl}(methyl)amino)pentyl](methyl)amino}phenyl)diazen-1-yl]-1,3-thiazol-3-iums 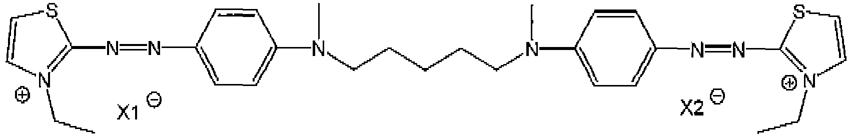
- Salzen des 3-Ethyl-2-(2-{4-[ethyl({3-[ethyl({4-[2-(3-ethyl-1,3-thiazol-3-ium-2-yl)diazen-1-yl]phenyl})amino]propyl})amino]phenyl}diazen-1-yl)-1,3-thiazol-3-iums 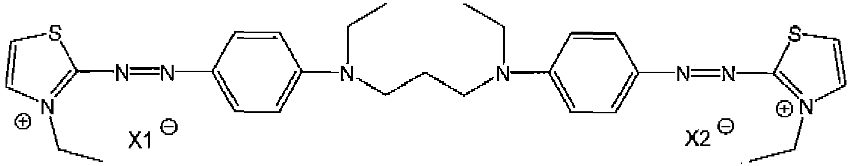
- Salzen des 3-Ethyl-2-(2-{4-[ethyl({4-[ethyl({4-[2-(3-ethyl-1,3-thiazol-3-ium-2-yl)diazen-1-yl]phenyl})amino]butyl})amino]phenyl}diazen-1-yl)-1,3-thiazol-3-iums 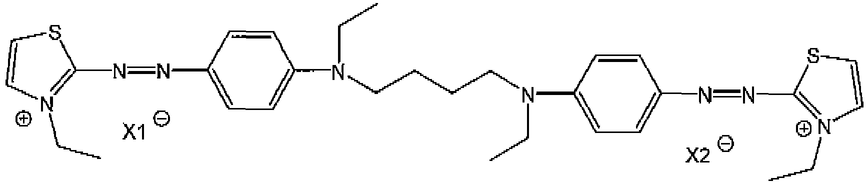
- Salzen des 3-Ethyl-2-(2-{4-[ethyl({5-[ethyl({4-[2-(3-ethyl-1,3-thiazol-3-ium-2-yl)diazen-1-yl]phenyl})amino]pentyl})amino]phenyl}diazen-1-yl)-1,3-thiazol-3-iums

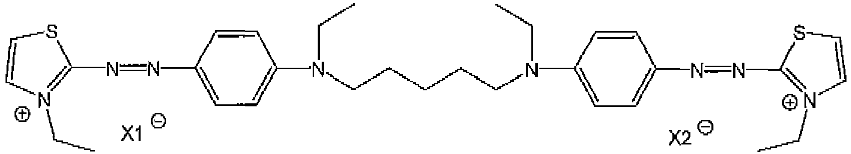

Bei den vorgenannten Verbindungen handelt es sich um dikationische dimere Farbstoffe, wobei das organische Dikation durch die beiden Anionen X1- und X2- neutralisiert wird. Bei den Anionen X1- und X2 kann es sich jeweils um ein physiologisch verträgliches Anion, bevorzugt aus der Gruppe aus Chlorid, Bromid, lodid, Methylsulfat, Methylsulfonat, p-Toluolsulfonat, Acetat, Hydrogensulfat, ½ Sulfat oder ½ Tetrachlorozinkat handeln.

In einer besonders bevorzugten Ausführungsform ist ein erfindungsgemäßes Mittel zum Färben von keratinischen Fasern dadurch gekennzeichnet, dass es als Farbstoff der Formel (a) mindestens eine Verbindung enthält, die ausgewählt ist aus der Gruppe aus
- 3-Methyl-2-[2-(4-{[3-({4-[2-{3-methyl-1,3-thiazol-3-ium-2-yl)diazen-1-yl]phenyl}amino)propyl]amino}phenyl)diazen-1-yl]-1,3-thiazol-3-ium Dichlorid
- 3-Methyl-2-[2-(4-{[3-({4-[2-(3-methyl-11,3-thiazol-3-ium-2-yl)diazen-1-yl]phenyl}amino)propyl]amino}phenyl)diazen-1-yl]-1,3-thiazol-3-ium Dibromid
- 3-Methyl-2-[2-(4-{[3-({4-[2-(3-methyl-1,3-thiazol-3-ium-2-yl)diazen-1-yl]phenyl}amino)propyl]amino}phenyl)diazen-1-yl]-1,3-thiazol-3-ium Sulfat
- 3-Methyl-2-[2-(4-{[3-({4-[2-(3-methyl-1,3-thiazol-3-ium-2-yl)diazen-1-yl]phenyl}amino)propyl]amino}phenyl)diazen-1-yl]-1,3-thiazol-3-ium Di(toluolsulfonat)
- 3-Methyl-2-[2-(4-{[3-({4-[2-(3-methyl-1,3-thiazol-3-ium-2-yl)diazen-1-yl]phenyl}amino)propyl]amino}phenyl)diazen-1-yl]-1,3-thiazol-3-ium Di(methylsulfat)
- 3-Methyl-2-[2-(4-{[3-({4-[2-(3-methyl-1,3-thiazol-3-ium-2-yl)diazen-1-yl]phenyl}amino)propyl]amino}phenyl)diazen-1-yl]-1,3-thiazol-3-ium Tetrarhlorozinkat
- 3-Methyl-2-[2-(4-{[4-({4-[2-(3-methyl-1,3-thiazol-3-ium-2-yl)diazen-1-yl]phenyl}amino)butyl]amino}phenyl)diazen-1-yl]-1,3-thiazol-3-ium Dichlorid
- 3-Methyl-2-[2-(4-{[4-({4-[2-(3-methy)-1,3-thiazol-3-ium-2-yl)diazen-1-yl]phenyl}amino)butyl]amino}phenyl)diazen-1-yl]-1,3-thiazol-3-ium Dibromid
- 3-Methyl-2-[2-(4-{[4-({4-[2-(3-methyl-1,3-thiazol-3-ium-2-yl)diazen-1-yl]phenyl}amino)butyl]amino}phenyl)diazen-1-yl]-1,3-thiazol-3-ium Sulfat
- 3-Methyl-2-[2-(4-{[4-({4-[2-(3-methyl-1,3-thiazol-3-ium-2-yl)diazen-1-yl]phenyl}amino)butyl]amino}phenyl)diazen-1-yl]-1,3-thiazol-3-ium Di(toluolsulfonat)
- 3-Methyl-2-[2-(4-{[4-({4-[2-(3-methy[-1,3-thiazol-3-ium-2-yl)diazen-1-yl]phenyl}amino)butyl]amino}phenyl)diazen-1-yl]-1,3-thiazol-3-ium Di(methylsulfat)
- 3-Methyl-2-[2-(4-{[4-({4-[2-(3-methyl-1,3-thiazol-3-ium-2-yl)diazen-1-yl]phenyl}amino)butyl]amino}phenyl)diazen-1-yl]-1,3-thiazol-3-ium Tetrachlorozinkat
- 3-Methyl-2-[2-(4-{[5-({4-[2-(3-methyl-1,3-thiazol-3-ium-2-yl)diazen-1-yl]phenyl}amino)pentyl]amino}phenyl)diazen-1-yl]-1,3-thiazol-3-ium Dichlorid
- 3-Methyl-2-[2-(4-{[5-({4-[2-(3-methyl-1,3-thiazol-3-ium-2-yl)diazen-1-yl]phenyl}amino)pentyl]amino}phenyl)diazen-1-yl]-1,3-thiazol-3-ium Dibromid
- 3-Methyl-2-[2-(4-{[5-({4-[2-(3-methyl-1,3-thiazol-3-ium-2-yl)diazen-1-yl]phenyl}amino)pentyl]amino}phenyl)diazen-1-yl]-1,3-thiazol-3-ium Sulfat
- 3-Methyl-2-[2-(4-{[5-({4-[2-(3-methyl-1,3-thiazol-3-ium-2-yl)diazen-1-yl]phenyl}amino)pentyl]amino}phenyl)diazen-1-yl]-1,3-thiazol-3-ium Di(toluolsulfonat)
- 3-Methyl-2-[2-(4-{[5-({4-[2-(3-methyl-1,3-thiazol-3-ium-2-yl)diazen-1-yl]phenyl}amino)pentyl]amino}phenyl)diazen-1-yl]-1,3-thiazol-3-ium (Di(methylsulfat)
- 3-Methyl-2-[2-(4-{[5-({4-[2-(3-methyl-1,3-thiazol-3-ium-2-yl)diazen-1-yl]phenyl}amino)pentyl]amino}phenyl)diazen-1-yl]-1,3-thiazol-3-ium Tetrachlorozinkat
- 3-Methyl-2-(2-{4-[methyl({3-[methyl({4-[2-(3-methyl-1,3-thiazol-3-ium-2-yl)diazen-1-yl]phenyl})amino]propyl})amino]phenyl}diazen-1-yl)-1,3-thiazol-3-ium Dichlorid
- 3-Methyl-2-(2-{4-[methyl({3-[methyl({4-[2-(3-methyl-1,3-thiazol-3-ium-2-yl)diazen-1-yl]phenyl})amino]propyl})amino]phenyl}diazen-1-yl)-1,3-thiazol-3-ium Dibromid
- 3-Methyl-2-(2-[4-[methy]({3-[methyl({4-[2-(3-methyl-1,3-thiazol-3-ium-2-yl)diazen-1-yl]phenyl})amino]propyl})amino]phenyl}diazen-1-yl)-1,3-thiazol-3-ium Sulfat
- 3-Methyl-2-(2-{4-[methyl({3-[methyl({4-[2-(3-methyl-1,3-thiazol-3-ium-2-yl)diazen-1-yl]phenyl})amino]propyl})amino]phenyl}diazen-1-yl)-1,3-thiazol-3-ium Di(toluolsulfonat)
- 3-Methyl-2-(2-{4-[methyl({3-[methyl({4-[2-(3-methyl-1,3-thiazol-3-ium-2-yl)diazen-1-yl]phenyl})amino]propyl})amino]phenyl}diazen-1-yl)-1,3-thiazol-3-ium Di(methylsulfat)
- 3-Methyl-2-(2-{4-[methyl({3-[methyl({4-[2-(3-methyl-1,3-thiazol-3-ium-2-yl)diazen-1-yl]phenyl})amino]propyl})amino]phenyl}diazen-1-yl)-1,3-thiazo]-3-ium Tetrachlorozinkat
- 3-Methyl-2-(2-{4-[methyl({4-[methyl({4-[2-(3-methyl-1,3-thiazol-3-ium-2-yl)diazen-1-yl]phenyl})amino]butyl})amino]phenyl}diazen-1-yl)-1,3-thiazol-3-ium Dichlorid
- 3-Methyl-2-{2-{4-[methyl({4-[methyl({4-[2-(3-methyl-1,3-thiazol-3-ium-2-yl)diazen-1-yl]phenyl})amino]butyl})amino]phenyl}diazen-1-yl)-1,3-thiazol-3-ium Dibromid
- 3-Methyl-2-(2-(4-[methyl({4-[methyl({4-[2-(3-methyl-1,3-thiazol-3-ium-2-yl)diazen-1-yl]phenyl})amino]butyl})amino]phenyl}diazen-1-yl)-1,3-thiazol-3-ium Sulfat
- 3-Methyl-2-(2-{4-[methyl({4-[methyl({4-[2-(3-methy]-1,3-thiazol-3-ium-2-yl)diazen-1-yl]phenyl})amino]butyl})amino]phenyl}diazen-1-yl)-1,3-thiazol-3-ium Di(toluolsulfonat)
- 3-Methyl-2-(2-{4-[methyl({4-[methyl({4-[2-(3-methyl-1,3-thiazol-3-ium-2-yl)diazen-1-yl]phenyl})amino]butyl})amino]phenyl)diazen-1-yl)-1,3-thiazol-3-ium Di(methylsulfat)
- 3-Methyl-2-(2-{4-[methyl({4-[methyl({4-[2-(3-methyl-1,3-thiazol-3-ium-2-yl)diazen-1-yl]phenyl})amino]butyl})amino]phenyl}diazen-1-yl)-1,3-thiazol-3-ium Tetrachlorozinkat
- 3-Methyl-2-(2-{4-[methyl({5-[methyl({4-[2-(3-methyl-1,3-thiazol-3-ium-2-yl)diazen-1-yl]phenyl})amino]pentyl})amino]phenyl}diazen-1-yl)-1,3-thiazol-3-ium Dichlorid
- 3-Methyl-2-(2-{4-[methyl({5-[methyl({4-[2-(3-methyl-1,3-thiazol-3-ium-2-yl)diazen-1-yl]phenyl})amino]pentyl})amino]phenyl}diazen-1-yl)-1,3-thiazol-3-ium Dibromid
- 3-Methyl-2-(2-{4-[methyl({5-[methyl({4-[2-(3-methyl-1,3-thiazol-3-ium-2-yl)diazen-1-yl]phenyl})amino]pentyl})amino]phenyl}diazen-1-yl)-1,3-thiazol-3-ium Sulfat
- 3-Methyl-2-(2-{4-[methyl({5-[methyl({4-[2-(3-methyl-1,3-thiazol-3-ium-2-yl)diazen-1-yl]phenyl})amino]pentyl})amino]phenyl}diazen-1-yl)-1,3-thiazol-3-ium Di(toluolsulfonat)
- 3-Methyl-2-(2-{4-[methyl({5-[methyl({4-[2-(3-methyl-1,3-thiazol-3-ium-2-yl)diazen-1-yl]phenyl})amino]pentyl})amino]phenyl}diazen-1-yl)-1,3-thiazol-3-ium Di(methylsulfat)
- 3-Methyl-2-(2-(4-[methyl({5-[methyl({4-[2-(3-methyl-1,3-thiazol-3-ium-2-yl)diazen-1-yl]phenyl})amino]pentyl})amino]phenyl}diazen-1-yl)-1,3-thiazol-3-ium Tetrachlorozinkat
- 2-(2-{4-[Ethyl({3-[ethyl({4-[2-(3-methyl-1,3-thiazol-3-uim-2-yl)diazen-1-yl]phenyl})amino]propyl})amino]phenyl}diazen-1-yl)-3-methyl-1,3-thiazol-3-ium Dichlorid
- 2-(2-{4-[Ethyl({3-[ethyl({4-[2-(3-methyl-1,3-thiazol-3-ium-2-yl)diazen-1-yl]phenyl})amino]propyl})amino]phenyl}diazen-1-yl)-3-methyl-1,3-thiazol-3-ium Dibromid
- 2-(2-{4-[Ethyl({3-[ethyl({4-[2-(3-methyl-1,3-thiazol-3-ium-2-yl)diazen-1-yl]phenyl})amino]propyl})amino]phenyl}diazen-1-yl)-3-methyl-1,3-thiazol-3-ium Sulfat
- 2-(2-{4-[Ethyl({3-[ethyl({4-[2-(3-methyl-1,3-thiazol-3-ium-2-yl)diazen-1-yl]phenyl})amino]propyl})amino]phenyl}diazen-1-yl)-3-methyl-1,3-thiazol-3-ium Di(toluolsulfonat)
- 2-(2-{4-[Ethyl({3-[ethyl({4-[2-(3-methyl-1,3-thiazol-3-ium-2-yl)diazen-1-yl]phenyl})amino]propyl})amino]phenyl}diazen-1-yl)-3-methyl-1,3-thiazol-3-ium Di(methylsulfat)
- 2-(2-{4-[Ethyl({3-[ethyl({4-[2-(3-methyl-1,3-thiazol-3-ium-2-yl)diazen-1-yl]phenyl})amino]propyl})amino]phenyl}diazen-1-yl)-3-methyl-1,3-thiazol-3-ium Tetrachlorozinkat
- 2-(2-{4-[Ethyl({4-[ethyl({4-[2-(3-methyl-1,3-thiazol-3-ium-2-yl)diazen-1-yl]phenyl})amino]butyl})amino]phenyl}diazen-1-yl)-3-methyl-1,3-thiazol-3-ium Dichlorid
- 2-(2-{4-[Ethyl({4-[ethyl({4-[2-(3-methyl-1,3-thiazol-3-ium-2-yl)diazen-1-yl]phenyl})amino]butyl})amino]phenyl}diazen-1-yl)-3-methyl-1,3-thiazol-3-ium Dibromid
- 2-(2-{4-[Ethyl({4-[ethyl({4-[2-(3-methyl-1,3-thiazol-3-ium-2-yl)diazen-1-yl]phenyl})amino]butyl})amino]phenyl)diazen-1-yl)-3-methyl-1,3-thiazol-3-ium Sulfat
- 2-(2-{4-[Ethyl({4-[ethyl({4-[2-(3-methyl-1,3-thiazol-3-ium-2-yl)diazen-1-yl]phenyl})amino]butyl})amino]phenyl}diazen-1-yl)-3-methyl-1,3-thiazol-3-ium Di(toluolsulfonat)
- 2-(2-{4-[Ethyl({4-[ethyl({4-[2-(3-methyl-1,3-thiazol-3-ium-2-yl)diazen-1-yl]phenyl})amino]butyl})amino]phenyl}diazen-1-yl)-3-methyl-1,3-thiazol-3-ium Di(methylsulfat)
- 2-(2-{4-[Ethyl({4-[ethyl({4-[2-(3-methyl-1,3-thiazol-3-ium-2-yl)diazen-1-yl]phenyl})amino]butyl})amino]phenyl}diazen-1-yl)-3-methyl-1,3-thiazol-3-ium Tetrachlorozinkat
- 2-(2-{4-[Ethyl({5-[ethyl({4-[2-(3-methyl-1,3-thiazol-3-ium-2-yl)diazen-1-yl]phenyl})amino]pentyl})amino]phenyl}diazen-1-yl)-3-methyl-1,3-thiazol-3-ium Dichlorid
- 2-{2-{4-[Ethyl({5-[ethyl({4-[2-(3-methyl-1,3-thiazol-3-ium-2-yl)diazen-1-yl]phenyl})amino]pentyl})amino]phenyl}diazen-1-yl)-3-methyl-1,3-thiazol-3-ium Dibromid
- 2-(2-{4-[Ethyl({5-[ethyl({4-[2-(3-methyl-1,3-thiazol-3-ium-2-yl)diazen-1-yl]phenyl})amino]pentyl})amino]phenyl}diazen-1-yl)-3-methyl-1,3-thiazol-3-ium Sulfat
- 2-(2-{4-[Ethyl({5-[ethyl({4-[2-(3-methy-1,3-thiazol-3-ium-2-yl)diazen-1-yl]phenyl})amino]pentyl})amino]phenyl}diazen-1-yl)-3-methyl-1,3-thiazol-3-ium Di(toluolsulfonat)
- 2-(2-{4-[Ethyl({5-[ethyl({4-[2-(3-methyl-1,3-thiazol-3-ium-2-yl)diazen-1-yl]phenyl})amino]pentyl})amino]phenyl}diazen-1-yl)-3-methyl-1,3-thiazol-3-ium Di(methylsulfat)
- 2-(2-{4-[Ethyl({4-[ethyl({4-[2-(3-methyl-1,3-thiazol-3-ium-2-yl)diazen-1-yl]phenyl})amino]pentyl})amino]phenyl}diazen-1-yl)-3-methyl-1,3-thiazol-3-ium Tetrachlorozinkat
- 3-Ethyl-2-[2-(4-{[3-({4-[2-(3-ethyl-1,3-thiazol-3-ium-2-yl)diazen-1-yl]phenyl}amino)propyl]amino}phenyl)diazen-1-yl]-1,3-thiazol-3-ium Dichlorid
- 3-Ethyl-2-[2-(4-{[3-({4-[2-(3-ethyl-1,3-thiazol-3-ium-2-yl)diazen-1-yl]phenyl)amino)propyl]amino}phenyl)diazen-1-yl]-1,3-thiazol-3-ium Dibromid
- 3-Ethyl-2-[2-(4-{[3-({4-[2-(3-ethyl-1,3-thiazol-3-ium-2-yl)diazen-1-yl]phenyl}amino)propyl]amino}phenyl)diazen-1-yl]-1,3-thiazol-3-ium Sulfat
- 3-Ethyl-2-[2-(4-{[3-({4-[2-(3-ethyl-1,3-thiazol-3-ium-2-yl)diazen-1-yl]phenyl}amino)propyl]amino}phenyl)diazen-1-yl]-1,3-thiazo]-3-ium Di(toluolsulfonat)
- 3-Ethyl-2-[2-(4-{[3-({4-[2-(3-ethyl-1,3-thiazol-3-ium-2-yl)diazen-1-yl]phenyl)amino)propyl]amino}phenyl)diazen-1-yl)-1,3-thiazol-3-ium Di(methylsulfat)
- 3-Ethyl-2-[2-(4-{[3-({4-[2-(3-ethyl-1,3-thiazol-3-ium-2-yl)diazen-1-yl]phenyl}amino)propyl]amino}pheny[)diazen-1-yl]-1,3-thiazol-3-ium Tetrachlorozinkat
- 3-Ethyl-2-[2-(4-{[4-({4-[2-(3-ethyl-1,3-thiazol-3-ium-2-yl)diazen-1-yl]phenyl}amino)butyl]amino}phenyl)diazen-1-yl]-1,3-thiazol-3-ium Dichlorid
- 3-Ethyl-2-[2-(4-{[4-({4-[2-(3-ethyl-1,3-thiazol-3-ium-2-yl)diazen-1-yl]phenyl}amino)butyl]amino}phenyl)diazen-1-yl]-1,3-thiazol-3-ium Dibromid
- 3-Ethyl-2-[2-(4-{[4-({4-[2-(3-ethyl-1,3-thiazol-3-ium-2-yl)diazen-1-yl]phenyl}amino)butyl]amino}phenyl)diazen-1-yl]-1,3-thiazol-3-ium Sulfat
- 3-Ethyl-2-[2-(4-{[4-({4-[2-(3-ethyl-1,3-thiazol-3-ium-2-yl)diazen-1-yl]phenyl}amino)butyl]amino}phenyl)diazen-1-yl]-1,3-thiazol-3-ium Di(toluolsulfonat)
- 3-Ethyl-2-[2-(4-{[4-({4-[2-(3-ethyl-1,3-thiazol-3-ium-2-yl)diazen-1-yl]phenyl}amino)butyl]amino}phenyl)diazen-1-yl]-1,3-thiazol-3-ium Di(methylsulfat)
- 3-Ethyl-2-[2-(4-{[4-({4-[2-(3-ethyl-1,3-thiazol-3-ium-2-yl)diazen-1-yl]phenyl}amino)butyl]amino}phenyl)diazen-1-yl]-1,3-thiazol-3-ium Tetrachlorozinkat
- 3-Ethyl-2-[2-(4-{[5-({4-[2-(3-ethyl-1,3-thiazol-3-ium-2-y])diazen-1-yl]phenyl}amino)pentyl]amino}phenyl)diazen-1-yl]-1,3-thiazol-3-ium Dichlorid
- 3-Ethyl-2-[2-(4-{[5-({4-[2-(3-ethy]-1,3-thiazol-3-ium-2-yl)diazen-1-yl]phenyl}amino)pentyl]amino}phenyl)diazen-1-yl]-1,3-thiazol-3-ium Dibromid
- 3-Ethyl-2-[2-(4-{[5-({4-[2-(3-ethyl-1,3-thiazol-3-ium-2-yl)diazen-1-yl]phenyl}amino)pentyl]amino}phenyl)diazen-1-yl]-1,3-thiazol-3-ium Sulfat
- 3-Ethyl-2-[2-(4-{[5-({4-[2-(3-ethyl-1,3-thiazol-3-ium-2-yl)diazen-1-yl]phenyl}amino)pentyl]amino}phenyl)diazen-1-yl]-1,3-thiazol-3-ium Di(toluolsulfonat)
- 3-Ethyl-2-[2-(4-{[5-([4-[2-(3-ethyl-1,3-thiazol-3-ium-2-yl)diazen-1-yl]phenyl}amino)pentyl]amino}phenyl)diazen-1-yl]-1,3-thiazol-3-ium Di(methylsulfat)
- 3-Ethyl-2-[2-(4-{[5-({4-[2-(3-ethyl-1,3-thiazol-3-ium-2-yl)diazen-1-yl]phenyl}amino)pentyl]amino]phenyl)diazen-1-yl]-1,3-thiazol-3-ium Tetrachlorozinkat
- 3-Ethyl-2-[2-(4-{[3-({4-[2-(3-ethyl-1,3-thiazol-3-ium-2-yl)diazen-1-yl]phenyl}(methyl)amino)propyl](methyl)amino}phenyl)diazen-1-yl]-1,3-thiazol-3-ium Dichlorid
- 3-Ethyl-2-[2-(4-{[4-({4-[2-(3-ethyl-1,3-thiazol-3-ium-2-yl)diazen-1-yl]phenyl}(methyl)amino)propyl](methyl)amino}phenyl)diazen-1-yl]-1,3-thiazol-3-ium Dibromid
- 3-Ethyl-2-[2-(4-{[3-({4-[2-(3-ethyl-1,3-thiazol-3-ium-2-yl)diazen-1-yl]phenyl}(methyl)amino)propyl](methyl)amino}phenyl)diazen-1-yl]-1,3-thiazol-3-ium Sulfat
- 3-Ethyl-2-[2-(4-{[3-({4-[2-(3-ethyl-1,3-thiazol-3-ium-2-yl)diazen-1-yl]phenyl}(methyl)amino)propyl]-(methyl)amino}phenyl)diazen-1-yl]-1,3-thiazol-3-ium Di(toluolsulfonat)
- 3-Ethyl-2-[2-(4-{[3-({4-[2-(3-ethyl-1,3-thiazol-3-ium-2-yl)diazen-1-yl]phenyl}-(methyl)amino)propyl](methyl)amino}phenyl)diazen-1-yl]-1,3-thiazol-3-ium Di(methylsulfat)
- 3-Ethyl-2-[2-(4-{[3-({4-[2-(3-ethyl-1,3-thiazol-3-ium-2-yl)diazen-1-yl]phenyl}-(methyl)amino)propyl](methyl)amino}phenyl)diazen-1-yl]-1,3-thiazol-3-ium Tetrachlorozinkat
- 3-Ethyl-2-[2-(4-{[4-({4-[2-(3-ethyl-1,3-thiazol-3-ium-2-yl)diazen-1-yl]phenyl}(methyl)amino)butyl](methyl)amino}phenyl)diazen-1-yl]-1,3-thiazo)-3-ium Dichlorid
- 3-Ethyl-2-[2-(4-{[4-({4-[2-(3-ethyl-1,3-thiazol-3-ium-2-yl)diazen-1-yl]phenyl}(methyl)amino)butyl](methyl)amino}phenyl)diazen-1-yl]-1,3-thiazol-3-ium Dibromid
- 3-Ethyl-2-[2-(4-{[4-({4-[2-(3-ethyl-1,3-thiazol-3-ium-2-yl)diazen-1-yl]phenyl}(methyl)amino)butyl](methyl)amino}phenyl)diazen-1-yl]-1,3-thiazol-3-ium Sulfat
- 3-Ethyl-2-[2-(4-{[4-({4-[2-(3-ethyl-1,3-thiazol-3-ium-2-yl)diazen-1-yl]phenyl}-(methyl)amino)butyl](methyl)amino}phenyl)diazen-1-yl]-1,3-thiazol-3-ium Di(toluolsulfonat)
- 3-Ethyl-2-[2-{4-{[4-({4-[2-(3-ethyl-1,3-thiazol-3-ium-2-yl)diazen-1-yl]phenyl}-(methyl)amino)butyl](methyl)amino}phenyl)diazen-1-yl]-1,3-thiazol-3-ium Di(methylsulfat)
- 3-Ethyl-2-[2-(4-{[4-({4-[2-(3-ethyl-1,3-thiazol-3-ium-2-yl)diazen-1-yl]phenyl}-(methyl)amino)butyl](methyl)amino}phenyl)diazen-1-yl]-1,3-thiazol-3-ium
- 3-Ethyl-2-[2-(4-{[5-({4-[2-(3-ethyl-1,3-thiazol-3-ium-2-yl)diazen-1-yl]phenyl}(methyl)amino)pentyl](methyl)amino}phenyl)diazen-1-yl]-1,3-thiazol-3-ium Dichlorid
- 3-Ethyl-2-[2-(4-{[5-({4-[2-(3-ethyl-1,3-thiazol-3-ium-2-yl)diazen-1-yl]phenyl}(methyl)amino)pentyl](methyl)amino}phenyl)diazen-1-yl]-1,3-thiazol-3-ium Dibromid
- 3-Ethyl-2-[2-(4-{[5-({4-[2-(3-ethyl-1,3-thiazol-3-ium-2-yl)diazen-1-yl]phenyl}(methyl)amino)pentyl](methyl)amino}phenyl)diazen-1-yl]-1,3-thiazol-3-ium Sulfat
- 3-Ethyl-2-[2-(4-{[5-({4-[2-{3-ethyl-1,3-thiazol-3-ium-2-yl)diazen-1-yl]phenyl}-(methyl)amino)pentyl](methyl)amino}phenyl)diazen-1-yl]-1,3-thiazol-3-ium Di(toluolsulfonat)
- 3-Ethyl-2-[2-(4-{[5-({4-[2-(3-ethyl-1,3-thiazol-3-ium-2-yl)diazen-1-yl]phenyl}-(methyl)amino)pentyl](methyl)amino}phenyl)diazen-1-yl]-1,3-thiazol-3-ium Di(methylsulfat)
- 3-Ethyl-2-[2-(4-{[5-({4-[2-(3-ethyl-1,3-thiazol-3-ium-2-yl)diazen-1-yl]phenyl}-(methyl)amino)pentyl](methyl)amino}phenyl)diazen-1-yl]-1,3-thiazol-3-ium Tetrachlorozinkat
- 3-Ethyl-2-(2-{4-[ethyl({3-[ethyl({{4-[2-(3-ethyl-1,3-thiazol-3-ium-2-yl)diazen-1-yl]phenyl})amino]propyl})amino]phenyl}diazen-1-yl)-1,3-thiazol-3-ium Dichlorid
- 3-Ethyl-2-(2-{4-[ethyl({3-[ethyl({4-[2-(3-ethyl-1,3-thiazol-3-ium-2-yl)diazen-1-yl]phenyl})amino]propyl})amino]phenyl}diazen-1-yl)-1,3-thiazol-3-ium Dibromid
- 3-Ethyl-2-(2-{4-[ethyl({3-[ethyl({4-[2-(3-ethyl-1,3-thiazol-3-ium-2-yl)diazen-1-yl]phenyl})amino]propyl})amino]phenyl}diazen-1-yl)-1,3-thiazol-3-ium Sulfat
- 3-Ethyl-2-(2-{4-[ethyl({3-[ethyl({4-[2-(3-ethyl-1,3-thiazol-3-ium-2-yl)diazen-1-yl]phenyl})amino]propyl})amino]phenyl}diazen-1-yl)-1,3-thiazol-3-ium Di(toluolsulfonat)
- 3-Ethyl-2-(2-{4-[ethyl({3-[ethyl({4-[2-(3-ethyl-1,3-thiazol-3-ium-2-yl)diazen-1-yl]phenyl})amino]propyl})amino]phenyl}diazen-1-yl)-1,3-thiazol-3-ium Di(methylsulfat)
- 3-Ethyl-2-(2-{4-[ethyl({3-[ethyl({4-[2-(3-ethyl-1,3-thiazol-3-ium-2-yl)diazen-1-yl]phenyl})amino]propyl})amino]phenyl}diazen-1-yl)-1,3-thiazol-3-ium Tetrachlorozinkat
- 3-Ethyl-2-(2-{4-[ethyl({4-[ethyl({4-[2-(3-ethyl-1,3-thiazol-3-ium-2-yl)diazen-1-yl]phenyl})amino]butyl})amino]phenyl}diazen-1-yl)-1,3-thiazol-3-ium Dichlorid
- 3-Ethyl-2-(2-{4-[ethyl({4-[ethyl({4-[2-(3-ethyl-1,3-thiazol-3-ium-2-yl)diazen-1-yl]phenyl})amino]butyl})amino]phenyl}diazen-1-yl)-1,3-thiazol-3-ium Dibromid
- 3-Ethyl-2-(2-{4-[ethyl({4-[ethyl({4-[2-{3-ethyl-1,3-thiazol-3-ium-2-yl)diazen-1-yl]phenyl})amino]butyl})amino]phenyl}diazen-1-yl)-1,3-thiazol-3-ium Sulfat
- 3-Ethyl-2-(2-{4-[ethyl({4-[ethyl({4-[2-(3-ethyl-1,3-thiazol-3-ium-2-yl)diazen-1-yl]phenyl})amino]butyl})amino]phenyl}diazen-1-yl)-1,3-thiazol-3-ium Di(toluolsulfonat)
- 3-Ethyl-2-(2-{4-[ethyl({4-[ethyl({4-[2-(3-ethyl-1,3-thiazol-3-ium-2-yl)diazen-1-yl]phenyl})amino]butyl})amino]phenyl}diazen-1-yl)-1,3-thiazol-3-ium Di(methylsulfat)
- 3-Ethyl-2-{2-{4-[ethyl({4-[ethyl({4-[2-(3-ethyl-1,3-thiazol-3-ium-2-yl)diazen-1-yl]phenyl})amino]butyl})amino]phenyl}diazen-1-yl)-1,3-thiazol-3-ium Tetrachlorozinkat
- 3-Ethyl-2-(2-{4-[ethyl({5-[ethyl({4-[2-(3-ethyl-1,3-thiazol-3-ium-2-yl)diazen-1-yl]phenyl})amino]pentyl})amino]phenyl}diazen-1-yl)-1,3-thiazol-3-ium Dichlorid
- 3-Ethyl-2-(2-{4-[ethyl({5-tethyl({4-[2-(3-ethyl-1,3-thiazol-3-ium-2-yl)diazen-1-yl]phenyl})amino]pentyl})amino]phenyl}diazen-1-yl)-1,3-thiazol-3-ium Dibromid
- 3-Ethyl-2-(2-{4-[ethyl({5-[ethyl({4-[2-(3-ethyl-1,3-thiazol-3-ium-2-yl)diazen-1-yl]phenyl})amino}pentyl})amino]phenyl}diazen-1-yl)-1,3-thiazol-3-ium Sulfat
- 3-Ethyl-2-(2-{4-[ethyl({5-[ethyl({4-[2-(3-ethyl-1,3-thiazol-3-ium-2-yl)diazen-1-yl]phenyl})amino]pentyl})amino]phenyl}diazen-1-yl)-1,3-thiazol-3-ium Di(toluolsulfonat)
- 3-Ethyl-2-(2-{4-[ethyl({5-[ethyl({4-[2-(3-ethyl-1,3-thiazol-3-ium-2-yl)diazen-1-yl]phenyl})amino]pentyl})amino]phenyl}diazen-1-yl)-1,3-thiazol-3-ium Di(methylsulfat)
- 3-Ethyl-2-(2-{4-[ethyl({5-[ethyl({4-[2-(3-ethyl-1,3-thiazol-3-ium-2-yl)diazen-1-yl]phenyl})amino]pentyl})amino]phenyl}diazen-1-yl)-1,3-thiazol-3-ium Tetrachlorozinkat

Im Rahmen der zu dieser Erfindung führenden Arbeiten hat sich herausgestellt, dass mit den direktziehenden Farbstoffen (a) der Formel (I) besonders dann intensive Färbungen im Blaubereich erzielt werden konnten, wenn
- R1, R4: unabhängig voneinander für eine Methylgruppe oder eine Ethylgruppe stehen,
- R2, R3: unabhängig voneinander für ein Wasserstoffatom, für eine Methylgruppe oder für eine Ethylgruppe stehen,
- R5, R6, R7, R8: jeweils für ein Wasserstoffatom stehen
- Q: für eine Gruppierung der Formel (II) steht,

*-(CH₂)n-* (II)
und
- n: für die Zahl 3 oder 4 steht.

Explizit ganz besonders bevorzugt sind daher erfindungsgemäße Mittel zum Färben von keratinischen Fasern, welche mindestens einen Farbstoff (a) der Formel (I) enthalten, der ausgewählt ist aus der Gruppe aus
- Salzen des 3-Methyl-2-[2-(4-{[3-({4-[2-(3-methyl-1,3-thiazol-3-ium-2-yl)diazen-1-yl]phenyl}amino)propyl]amino}phenyl)diazen-1-yl]-1,3-thiazol-3-iums 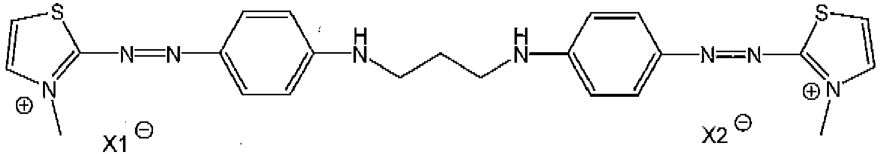
- Salzen des 3-Methyl-2-[2-(4-{[4-({4-[2-(3-methyl-1,3-thiazol-3-ium-2-yl)diazen-1-yl]phenyl}amino)butyl]amino}phenyl)diazen-1-yl]-1,3-thiazol-3-iums 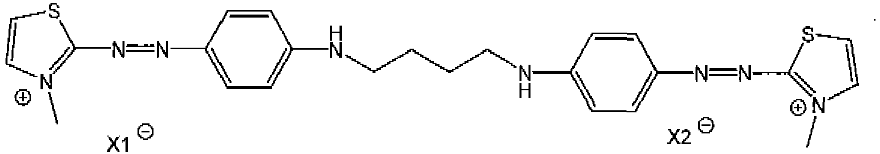
- Salzen des 3-Methyl-2-(2-{4-[methyl({3-[methyl({4-[2-(3-methyl-1,3-thiazol-3-ium-2-yl)diazen-1-yl]phenyl})amino]propyl})amino]phenyl}diazen-1-yl)-1,3-thiazol-3-iums 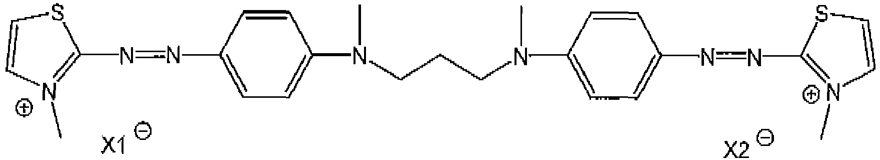
- Salzen des 3-Methyl-2-(2-{4-[methyl({4-[methyl({4-[2-(3-methyl-1,3-thiazol-3-ium-2-yl)diazen-1-yl]phenyl})amino]butyl})amino]phenyl}diazen-1-yl)-1,3-thiazol-3-iums 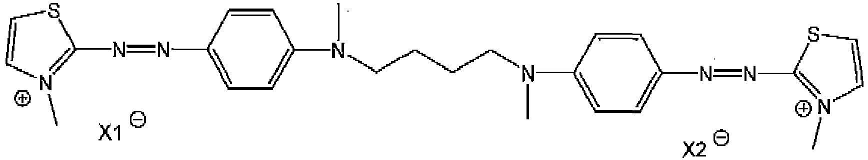
- Salzen des 2-(2-{4-[Ethyl({3-[ethyl({4-[2-(3-methyl-1,3-thiazol-3-ium-2-yl)diazen-1-yl]phenyl})amino]propyl})amino]phenyl}diazen-1-yl)-3-methyl-1,3-thiazol-3-iums 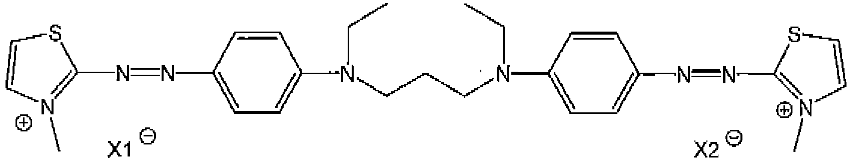
- Salzen des 2-(2-{4-[Ethyl({4-[ethyl({4-[2-(3-methyl-1,3-thiazol-3-ium-2-yl)diazen-1-yl]phenyl})amino]butyl})amino]phenyl}diazen-1-yl)-3-methyl-1,3-thiazol-3-iums 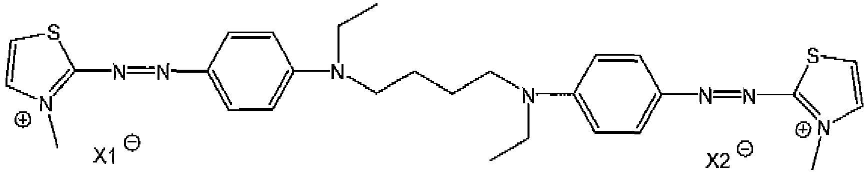
- Salzen des 3-Ethyl-2-[2-(4-{[3-({4-[2-(3-ethyl-1,3-thiazol-3-ium-2-yl)diazen-1-yl]phenyl}amino)propyl]amino}phenyl)diazen-1-yl]-1,3-thiazol-3-iums 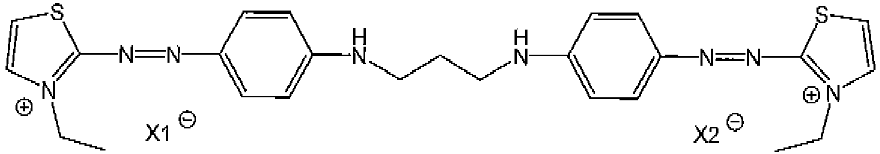
- Salzen des 3-Ethyl-2-[2-(4-{[4-({4-[2-(3-ethyl-1,3-thiazol-3-ium-2-yl)diazen-1-yl]phenyl}amino)butyl]amino}phenyl)diazen-1-yl]-1,3-thiazol-3-iums 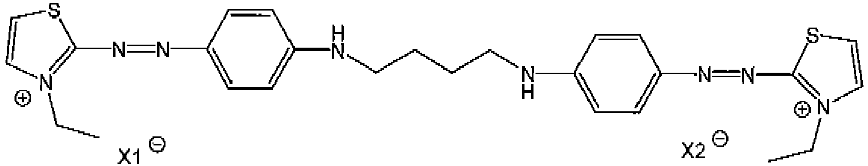
- Salzen des 3-Ethyl-2-[2-(4-{[3-({4-[2-{3-ethyl-1,3-thiazol-3-ium-2-yl)diazen-1-yl]phenyl}(methyl)amino)propyl](methyl)amino}phenyl)diazen-1-yl]-1,3-thiazol-3-iums 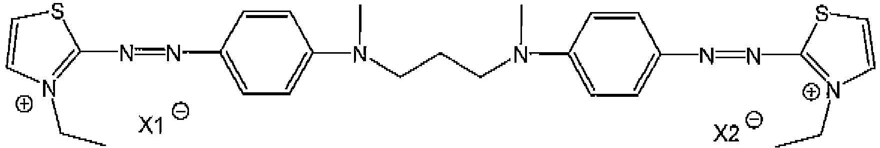
- Salzen des 3-ethyl-2-[2-(4-([4-({4-[2-(3-ethyl-1,3-thiazol-3-ium-2-yl)diazen-1-yl]phenyl}(methyl)amino)butyl](methyl)amino}phenyl)diazen-1-yl]-1,3-thiazol-3-iums 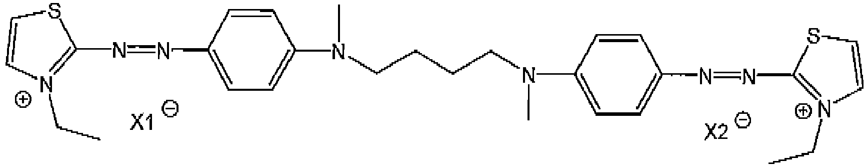
- Salzen des 3-Ethyl-2-(2-{4-[ethyl({3-[ethyl({4-[2-(3-ethyl-1,3-thiazol-3-ium-2-yl)diazen-1-yl]phenyl})amino]propyl})amino]phenyl}diazen-1-yl)-1,3-thiazol-3-iums 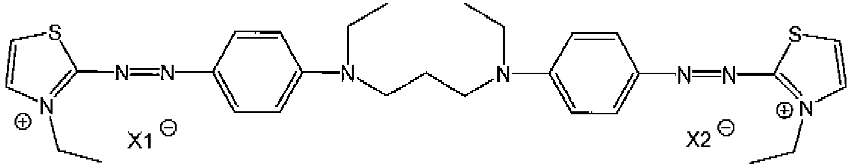
- Salzen des 3-Ethyl-2-(2-{4-[ethyl({4-[ethyl({4-[2-(3-ethyl-1,3-thiazol-3-ium-2-yl)diazen-1-yl]phenyl})amino]butyl})amino]phenyl}diazen-1-yl)-1,3-thiazol-3-iums

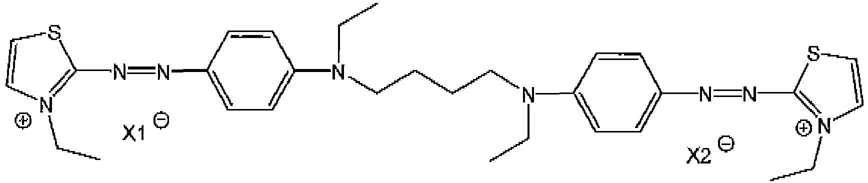

Die erfindungsgemäßen Mittel zum Färben von keratinischen Fasern enthalten den oder die direktziehenden Farbstoffe der Formel (I) vorzugsweise in einer Gesamtmenge von 0,01 bis 4,5 Gew.-%, bevorzugt von 0,05 bis 2,8 Gew.-%, weiter bevorzugt von 0,1 bis 2,2 Gew.-% und besonders bevorzugt von 0,2 bis 1,2 Gew.-% enthält. Die Mengenangabe in Gewichtsprozent bezieht sich hierbei auf die Gesamtmenge aller im Mittel enthaltenen Verbindungen der Formel (I), die zum Gesamtgewicht des Mittels in Relation gesetzt wird.

In einer weiteren bevorzugten Ausführungsform ist ein erfindungsgemäßes Mittel zum Färben von keratinischen Fasern daher dadurch gekennzeichnet, dass es - bezogen auf das Gesamtgewicht des Mittels - einen oder mehrere direktziehende Farbstoffe (a) der Formel (I) in einer Gesamtmenge von 0,01 bis 4,5 Gew.-%, bevorzugt von 0,05 bis 2,8 Gew.-%, weiter bevorzugt von 0,1 bis 2,2 Gew.-% und besonders bevorzugt von 0,2 bis 1,2 Gew.-% enthält.

Die Farbstoffe der allgemeinen Formel (I) können beispielsweise nach einem Verfahren hergestellt werden, wie es in WO 2002/100369 A2 beschrieben ist.

So kann beispielsweise das Edukt 2-Aminothiazol in konzentrierter Schwefelsäure mit Nitrosylschwefelsäure in das Diazoniumion überführt werden:

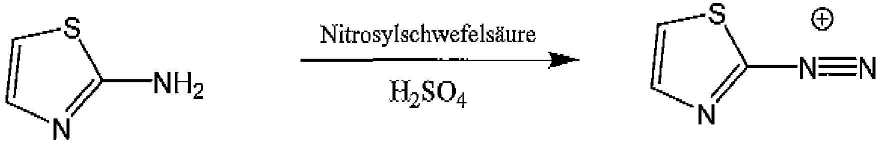

Das reaktive Diazoniumion geht dann mit dimeren Anilin-Derivaten eine doppelte Azokupplungsreaktion ein:

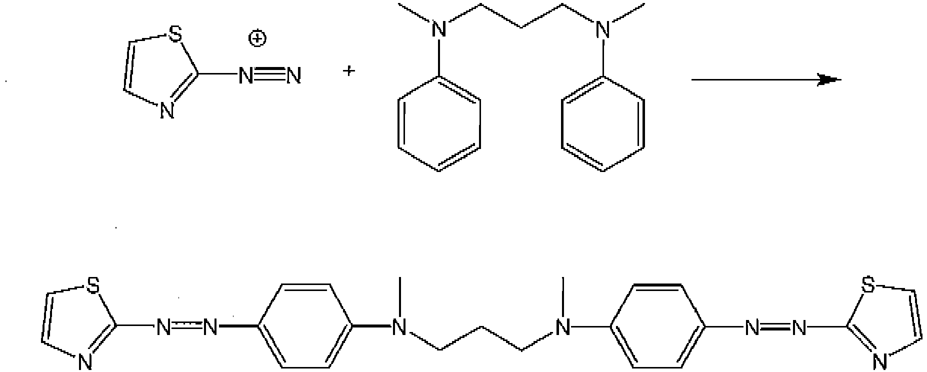

Der in der Azokupplungsreaktion entstehende neutrale dimere Farbstoff kann dann schließlich mit Quaternierungsmitteln doppelt quaterniert werden. Die Quaternierungsreaktion wird bevorzugt in einem polaren aprotischen Lösungsmittel (wie beispielsweise DMSO, DMF etc.) durchgeführt. Als Quaternierungsmittel kommen zum Beispiel Dimethylsulfat, Methyliodid oder p-Toluolsulfonat in Frage.

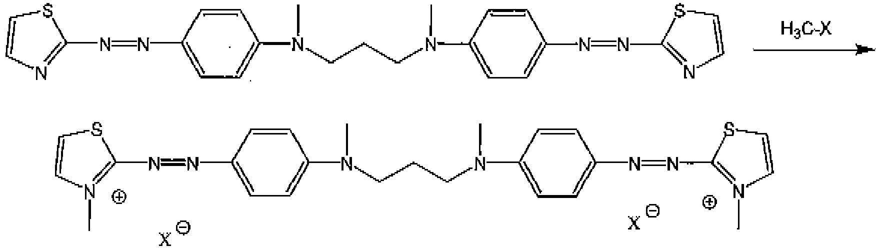

Als zweite erfindungswesentliche Komponente (b) enthalten die Mittel zum Färben von keratinischen Fasern mindestens ein anionisches Tensid.

Tenside sind amphiphile (bifunktionelle) Verbindungen, die aus mindestens einem hydrophoben und mindestens einem hydrophilen Molekülteil bestehen. Bei dem hydrophoben Rest handelt es sich bevorzugt eine Kohlenwasserstoffkette mit 8-24 Kohlenstoff-Atomen, die gesättigt oder ungesättigt, linear oder verzweigt sein kann. Besonders bevorzugt ist diese C₆-C₂₄-Alkylkette linear.

Bei anionischen Tensiden umfasst der hydrophile Molekülteil eine negativ geladene hydrophile Kopfgruppe. Bei der negativ geladenen hydrophilen Kopfgruppe kann es sich beispielsweise um eine Carbonsäuregruppe bwz. das Salz einer Carbonsäuregruppe, eine Sulfonsäuregruppe bzw. das Salz der Sulfonsäuregruppe, eine Schwefelsäureestergruppierung bzw. das Salz hiervon, eine Phosphonsäuregruppe bzw. das Salz der Phosphonsäuregruppe, oder eine Phosphorsäureestergruppierung bzw. das Salz hiervon handeln.

Üblicherweise umfasst das erfindungsgemäße kosmetische Mittel einen wässrigen Träger. In wässriger Lösung liegen die vorgenannten hydrophilen Kopfgruppen des anionischen Tensids - wie beispielsweise die Carbonsäure und die Salze der Carbonsäuren - in einem Gleichgewicht vor, dessen Lage vom pH-Wert des Mittels mitbestimmt wird. Wird als anionisches Tensid somit beispielsweise eine Fettsäure eingesetzt, so liegt ein geringer Teil der Fettsäure in wässriger Lösung in Form der protonierten Fettsäure vor, wohingegen der Großteil der Fettsäure in wässriger Lösung deprotoniert und auf diesem Wege in das Salz der Fettsäure überführt wird. Aus diesem Grund umfasst die Definition eines anionischen Tensid auch ein Tenside mit einer - noch protonierten - Säuregruppe.

Ein anionisches Tensid (b) im Sinne der vorliegenden Erfindung enthält keine kationischen Gruppierungen, d.h. zwitterionische Tenside sind von der Definition eines anionischen Tensids nicht mit umfasst.

Erfindungsgemäße anionische Tenside sind demnach gekennzeichnet durch die Anwesenheit einer wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder
Polyglykolether-Gruppen, Ester-, Ether- und Amidgruppen sowie Hydroxylgruppen enthalten sein.

Typische Beispiele für anionische Tenside sind Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, Acyllactylate, Acyltartrate, Acylglutamate, Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten. können diese eine konventionelle, vorzugsweise jedoch eine eingeengte Homologenverteilung aufweisen.

Beispiele für erfindungsgemäße anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(OH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe, die durch Veresterung von Fettsäuren mit dem Natriumsalz der 2-Hydroxyethan-sulfonsäure (Isethionsäure) zugänglich sind. Wenn man für diese Veresterung Fettsäuren mit 8 bis 24 C-Atomen, also z. B. Laurin-, Myristin-, Palimitin- oder Stearinsäure oder auch technische Fettsäurefraktionen, z. B. die aus Kokosfettsäure erhältliche C₁₂ - C₁₈-Fettsäurefraktion einsetzt, erhält man die erfindungsgemäß bevorzugt geeigneten C₁₂ - C₁₆-Acylisethionate,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen. Sulfobernsteinsäuremono- und -dialkylester können durch Umsetzung von Maleinsäureanhydrid mit einem Fettalkohol mit 8 - 24 C-Atomen zum Maleinsäuremonoester des Fettalkohols und Weiterreaktion mit Natriumsulfit zum Sulfobernsteinsäureester hergestellt werden. Besonders geeignete Sulfobernsteinsäureester leiten sich von Fettalkoholfraktionen mit 12 - 18 C-Atomen ab, wie sie z. B. aus Kokosfettsäure oder Kokosfettsäuremethylester durch Hydrierung zugänglich sind,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
- Hydroxysulfonate im wesentlichen entsprechend mindestens einer der beiden folgenden Formeln oder deren Mischungen sowie deren salzen, CH₃-(CH₂)_{y}-CHOH-(CH₂)ₚ-(CH-SO₃M)-(CH₂)_{z}-CH₂-O-(CₙH₂ₙO)ₓ-H, und/oder CH₃-(CH₂)_{y}-(CH-SO₃M)-(CH₂)ₚ-CHOH-(CH₂)₂-CH₂-O-(CₙH₂ₙO)ₓ-H wobei in beiden Formeln y und z = 0 oder ganze Zahlen von 1 bis 18, p = 0, 1 oder 2 und die Summe (y+z+p) eine Zahl von 12 bis 18, x = 0 oder eine Zahl von 1 bis 30 und n eine ganze Zahl von 2 bis 4 sowie M = H oder Alkali-, insbesondere Natrium, Kalium, Lithium, Erdalkali- , insbesondere Magnesium, Calcium, Zink und/oder einem Ammoniumion, welches gegebenenfalls substituiert sein kann, insbesondere Mono-, Di-, Tri- oder Tetraammoniumionen mit C1 bis C4 Alkyl-, Alkenyl- oder Arylresten,
- sulfatierte Hydroxyalkylpolyethylen- und/oder Hydroxyalkylenpropylenglykolether der Formel R¹-(CHOSO₃M)-CHR³-(OCHR⁴-CH₂)n-OR², mit R¹, einem linearen Alkylrest mit 1 bis 24 C-Atomen, R² für einen linearen oder verzweigten, gesättigten Alkylrest mit 1 bis 24 C-Atomen, R³ für Wasserstoff oder einen linearen Alkylrest mit 1 bis 24 C-Atomen, R⁴ für Wasserstoff oder einen Methylrest und M für Wasserstoff, Ammonium, Alkylammonium, Alkanolammonium, worin die Alkyl- und Alkanolreste je 1 bis 4 C-Atome aufweisen, oder ein Metallatom ausgewählt aus Lithium, Natrium, Kalium, Calcium oder Magnesium und n für eine Zahl im Bereich von 0 bis 12 stehen und weiterhin die Gesamtzahl der in R¹ und R³ enthaltenen C-Atome 2 bis 44 beträgt,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel, R¹(OCH₂CH₂)ₙ-O-(PO-OX)-OR²,
   in der R¹ bevorzugt für einen aliphatischen Kohlenwasserstoffrest mit 8 bis 30 Kohlenstoffatomen, R² für Wasserstoff, einen Rest (CH₂CH₂O)ₙR² oder X, n für Zahlen von 1 bis 10 und X für Wasserstoff, ein Alkali- oder Erdalkalimetall oder NR³R⁴R⁵R⁶, mit R³ bis R⁶ unabhängig voneinander stehend für Wasserstoff oder einen C₁ bis C₄ - Kohlenwasserstoffrest, steht,
- sulfatierte Fettsäurealkylenglykolester der Formel RCO(AlkO)ₙSO₃M
   in der RCO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Metall steht, wie Alkalimetall, insbesondere Natrium, Kalium, Lithium, Erdalkalimetall, insbesondere Magnesium, Calcium, Zink, oder Ammoniumion, wie ⁺NR³R⁴R⁵R⁶, mit R³ bis R⁶ unabhängig voneinander stehend für Wasserstoff oder einen C1 bis C4 - Kohlenwasserstoffrest,
- Monoglyceridsulfate und Monoglyceridethersulfate der Formel R⁸OC-(OCH₂CH₂)ₓ-OCH₂-[CHO(CH₂CH₂O)yH]-CH₂O(CH₂CH₂O)_{z}-SO₃X,
   in der R⁶CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate eingesetzt, in der R⁸CO für einen linearen Acylrest mit 8 bis 18 Kohlenstoffatomen steht,
- Amidethercarbonsäuren, R¹-CO-NR²-CH₂CH₂-O-(CH₂CH₂O)ₙCH₂COOM, mit R¹ als geradkettigen oder verzweigten Alkyl- oder Alkenylrest mit einer Zahl an Kohlenstoffatomen in der Kette von 2 bis 30, n steht für eine ganze Zahl von 1 bis 20 und R² steht für Wasserstoff, einen Methyl-, Ethyl-, Propyl-, Isopropyl-, n-Butyl-, t-Butyl- oder iso-Butylrest und M steht für Wasserstoff oder ein Metall wie Alkalimetall, insbesondere Natrium, Kalium, Lithium, Erdalkalimetall, insbesondere Magnesium, Calcium, Zink, oder ein Ammoniumion, wie ⁺NR³R⁴R⁵R⁶, mit R³ bis R⁶ unabhängig voneinander stehend für Wasserstoff oder einen C1 bis C4 - Kohlenwasserstoffrest. Derartige Produkte sind beispielsweise von der Firma Chem-Y unter der Produktbezeichnung Akypo® erhältlich, und
- Acylglutamate der Formel XOOC-CH2CH2CH(C(NH)OR)-COOX, in der RCO für einen linearen oder verzweigten Acylrest mit 6 bis 22 Kohlenstoffatomen und 0 und/oder 1, 2 oder 3 Doppelbindungen und X für Wasserstoff, ein Alkali- und/oder Erdalkalimetall, Ammonium, Alkylammonium, Alkanolammonium oder Glucammonium steht.

Die Behandlung von keratinischen Fasern mit Mitteln, die (a) mindestens einen direktiehenden Farbstoff der Formel (I) und (b) mindestens ein anionisches Tensid enthielten, führte zu intensiven Färbungen in attraktiven, reinen Blaunuancen ohne Rotanteil. Hierbei hat sich überraschenderweise gezeigt, dass das Farbaufzugsvermögen durch Einsatz eines oder mehrere spezieller anionischer Tenside noch weiter optimiert werden konnte. Besonders intensive Blaufärbungen wurden erhalten, wenn die Farbstoffe (a) der Formel (I) mit mindestens einem anionischen Tensid (b) aus der Gruppe der
- linearen und verzweigten Fettsäuren mit 8 bis 30 C-Atomen,
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe, die durch Veresterung von Fettsäuren mit dem Natriumsalz der 2-Hydroxyethan-sulfonsäure (Isethionsäure) zugänglich sind. Wenn man für diese Veresterung Fettsäuren mit 8 bis 24 C-Atomen, also z. B. Laurin-, Myristin-, Palimitin- oder Stearinsäure oder auch technische Fettsäurefraktionen, z. B. die aus Kokosfettsäure erhältliche C₁₂ - C₁₆-Fettsäurefraktion einsetzt, erhält man die erfindungsgemäß bevorzugt geeigneten C₁₂ - C₁₆-Acylisethionate,
- Amidethercarbonsäuren, R¹-CO-NR²-CH₂CH₂-O-(CH₂CH₂O)ₙCH₂COOM, mit R¹ als geradkettigen oder verzweigten Alkyl- oder Alkenylrest mit einer Zahl an Kohlenstoffatomen in der Kette von 2 bis 30, n steht für eine ganze Zahl von 1 bis 20 und R² steht für Wasserstoff, einen Methyl-, Ethyl-, Propyl-, Isopropyl-, n-Butyl-, t-Butyl- oder iso-Butylrest und M steht für Wasserstoff oder ein Metall wie Alkalimetall, insbesondere Natrium, Kalium, Lithium, Erdalkalimetall, insbesondere Magnesium, Calcium, Zink, oder ein Ammoniumion, wie ⁺NR³R⁴R⁵R⁶, mit R³ bis R⁶ unabhängig voneinander stehend für Wasserstoff oder einen C1 bis C4 - Kohlenwasserstoffrest. Derartige Produkte sind beispielsweise von der Firma Chem-Y unter der Produktbezeichnung Akypo® erhältlich, und
- Acylglutamate der Formel XOOC-CH2CH2CH(C(NH)OR)-COOX, in der RCO für einen linearen oder verzweigten Acylrest mit 6 bis 22 Kohlenstoffatomen und 0 und/oder 1, 2 oder 3 Doppelbindungen und X für Wasserstoff, ein Alkali- und/oder Erdalkalimetall, Ammonium, Alkylammonium, Alkanolammonium oder Glucammonium steht.

In einer besonders bevorzugten Ausführungsform ist ein erfindungsgemäßes Mittel daher dadurch gekennzeichnet, dass es (b) mindestens ein anionisches Tensid enthält, das ausgewählt ist aus den
- linearen und verzweigten Fettsäuren mit 8 bis 30 C-Atomen,
- Ethercarbonsäuren der Formel R-O-(CH₂-CH₂O)ₓ-CH₂-COOM, in der
   - R: für eine lineare Alkylgruppe mit 8 bis 30 C-Atomen steht,
   - X: für eine ganze Zahl von 0 bis 16 steht,
   - M: für Wasserstoff oder ein Metall wie Alkalimetall, insbesondere Natrium, Kalium, Lithium, Erdalkalimetall, insbesondere Magnesium, Calcium, Zink, oder ein Ammoniumion (NH4⁺) steht,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe, die durch Veresterung von C₈-C₂₄-Fettsäuren mit dem Natriumsalz der 2-Hydroxyethan-sulfonsäure (Isethionsäure) zugänglich sind,
- Amidethercarbonsäuren, R¹-CO-NR²-CH₂CH₂-O-(CH₂CH₂O)_{y}CH₂COOM, wobei
   - R¹: für eine C2-C30-Alkylgruppe steht,
   - y: für eine ganze Zahl von 1 bis 20 steht,
   - R²: für Wasserstoff, einen Methyl-, Ethyl-, Propyl-, Isopropyl-, n-Butyl-, t-Butyl- oder iso-Butylrest steht, und
   - M: für Wasserstoff oder ein Metall wie Alkalimetall, insbesondere Natrium, Kalium, Lithium, Erdalkalimetall, insbesondere Magnesium, Calcium, Zink, oder ein Ammoniumion (NH4⁺) steht.

Ein besonders gutes Farbaufzugsvermögen konnte beobachtet werden, wenn als Aniontensid (b) mindestens eine Ethercarbonsäuren der Formel R-O-(CH2-CH2O)x-CH2-COOH oder ihr Salz eingesetzt wurde. Der Einsatz von - gegebenenfalls ethoxylierten - Ethercarbonsäuren ist daher explizit ganz besonders bevorzgut.

In einer explizit ganz besonders bevorzugten Ausführungsform ist ein erfindungsgemäßes Mittel daher dadurch gekennzeichnet, dass als anionisches Tensid (b) mindestens eine Ethercarbonsäure der Formel (B1) enthält

R-O-(CH₂-CH₂O)x-CH2-COOM (B1),

wobei
- R: für eine lineare Alkylgruppe mit 8 bis 30 C-Atomen steht,
- X: für eine ganze Zahl von 0 bis 16 steht,
- M: für Wasserstoff oder ein Metall wie ein Alkalimetall, insbesondere Natrium, Kalium, Lithium, ein Erdalkalimetall, insbesondere ½ Magnesium, ½ Calcium, ½ Zink, oder ein Ammoniumion (NH4⁺) steht.

Weiterhin besonders bevorzugt steht x für die Zahlen 5, 6 oder 7.

In einer weiterhin ganz besonders bevorzugten Ausführungsform ist ein erfindungsgemäßes Mittel daher dadurch gekennzeichnet, dass als anionisches Tensid (b) mindestens eine Ethercarbonsäure der Formel (B1) enthält

R-O-(CH₂-CH₂O)x-CH2-COOM (B1),

wobei
- R: für eine lineare Alkylgruppe mit 8 bis 30 C-Atomen steht,
- x: für die ganze Zahl 5 bis 11 steht,
- M: für Wasserstoff oder ein Metall wie ein Alkalimetall, insbesondere Natrium, Kalium, Lithium, ein Erdalkalimetall, insbesondere ½ Magnesium, ½ Calcium, ½ Zink, oder ein Ammoniumion (NH4⁺) steht.

Ganz besonders bevorzugte anionische Tenside der Formel (B1) sind beispielsweise unter den Handelsnamen
- Akypo Soft 45 HP der Firma Kao (Natrium Laureth-6 Carboxylat)
- Akypo Soft 45 NV der Firma Kao (Natrium Laureth-5 Carboxylat)
- Akypo RLM 100 NV der Firma Kao (Natrium Laureth-11 Carboxylat)
erhältlich.

Bevorzugt enthalten die erfindungsgemäßen Mittel zum Färben von keratinischen Fasern ein oder mehrere anionische Tenside (b) in einer Gesamtmenge von 0,05 bis 4,5 Gew.-%, bevorzugt von 0,1 bis 3,1 Gew.-%, weiter bevorzugt von 0,15 bis 2,5 Gew.-% und ganz besonders bevorzugt von 0,2 bis 0,9 Gew.-%. Hierbei sind die Angaben in Gew.-% auf die Gesamtmenge aller anionischen Tenside (b) bezogen, die zur Gesamtmenge des Färbemittels in Relation gesetzt werden.

In einer weiteren besonders bevorzugten Ausführungsform ist ein erfindungsgemäßes Mittel zum Färben von keratinischen Fasern daher dadurch gekennzeichnet, dass es - bezogen auf das Gesamtgewicht des Mittels - ein oder mehrere anionische Tenside (b) in einer Gesamtmenge von 0,05 bis 4,5 Gew.-%, bevorzugt von 0,1 bis 3,1 Gew.-%, weiter bevorzugt von 0,15 bis 2,5 Gew.-% und ganz besonders bevorzugt von 0,2 bis 0,9 Gew.-% enthält.

Wie zuvor beschrieben hat sich gezeigt, dass durch Variation des bzw. der anionischen Tenside (b) das Farbaufzugsvermögen der direktziehenden Farbstoffe der Formel (I) beeinflusst werden kann.

Generell kann der Farbaufzug durch Einsatz mindestens eines anionischen Tensids verbessert werden. Der Einsatz mindestens einer - gegebenenfalls ethoxylierten - Ethercarbonsäure und/oder ihres Salzes, wie sie in Formel (B1) beschrieben ist, hat sich in diesem Zusammenhang als besonders vorteilhaft erwiesen. In diesem Zusammenhang wurde beobachtet, dass die Keratinfasern sich insbesondere dann in intensiven Blaunuancen färben ließen, wenn das bzw. die Ethercarbonsäuren (und/oder ihre Salze) als das hauptsächliche anionische Tensid im erfindungsgemäßen Mittel enthalten waren.

Mit anderen Worten waren die Färbeergebnisse dann besonders intensiv, wenn die erfindungsgemäßen Mitteln ein oder mehrere Ethercarbonsäuren der Formel (B1) enthielten, und wenn darüber hinaus alle weiteren eingesetzten anionischen Tenside nur in geringeren Mengen vorhanden waren. Der Einsatz der Ethercarbonsäure(n) der Formel (B1) als Haupt-Tensid lässt sich durch Angabe eines Gewichtsverhältnisses quantifizieren, welches die Gesamtmenge der im Mittel enthaltenen anionischen Tenside der Formel (B1) zur Gesamtmenge aller im Mittel enthaltenen anionischen Tenside (b) in Relation setzt.

In einer weiteren besonders bevorzugten Ausführungsform ist ein erfindungsgemäßes Mittel zum Färben von keratinischen Fasern daher dadurch gekennzeichnet, dass das Gewichtsverhältnis aus allen im Mittel enthaltenen anionischen Tensiden der Formel (B1) zur Gesamtmenge der im Mittel enthaltenen anionischen Tenside bei einem Wert von 0,5, bevorzugt von 0,6, weiter bevorzugt von 0,75 und besonders bevorzugt von 0,9 liegt.

Beispiel: Ein Mittel zum Färben von keratinischen Fasern enthält:
(a) 1,0 g 3-Methyl-2-[2-(4-{[3-({4-[2-(3-methyl-1,3-thiazol-3-ium-2-yl)diazen-1-yl]phenyl}amino)propyl]amino}phenyl)diazen-1-yl]-1,3-thiazol-3-ium Tetrachlorozinkat
(b) 0,23 g Natrium laureth-6 carboxylat (B1) und
(b) 0,10 g Natriumlaureth-Sulfat (2 EO)

Das Gewichtsverhältnis aus aus allen im Mittel enthaltenen anionischen Tensiden der Formel (B1) zur Gesamtmenge der im Mittel enthaltenen anionischen Tenside liegt bei einem Wert von [0,23 / (0,23 + 0,1)] = 0,70

Beispiel: Ein Mittel zum Färben von keratinischen Fasern enthält:
(a) 1,0 g 3-Methyl-2-[2-(4-{[3-({4-[2-(3-methyl-1,3-thiazol-3-ium-2-yl)diazen-1-yl]phenyl}amino)propyl]amino}phenyl)diazen-1-yl]-1,3-thiazol-3-iums Tetrachlorozinkat
(b) 0,23 g Natrium laureth-6 carboxylat (B1) und
   keine weiteren anionischen Tenside

Das Gewichtsverhältnis aus allen im Mittel enthaltenen anionischen Tensiden der Formel (B1) zur Gesamtmenge der im Mittel enthaltenen anionischen Tenside liegt bei einem Wert von [0,23 / 0,23] = 1,0

Weiterhin können die erfindungsgemäßen Mittel auch ein oder mehrere kationische Tenside enthalten.

Unter kationischen Tensiden werden Tenside, also grenzflächenaktive Verbindungen, mit jeweils einer oder mehreren positiven Ladungen verstanden. Kationische Tenside enthalten ausschließlich positive Ladungen. Üblicherweise sind diese Tenside aus einem hydrophoben Teil und einer hydrophilen Kopfgruppe aufgebaut, wobei der hydrophobe Teil in der Regel aus einem Kohlenwasserstoff-Gerüst (z.B. bestehend aus einer oder zwei linearen oder verzweigten Alkylketten) besteht, und die positive(n) Ladung(en) in der hydrophilen Kopfgruppe lokalisiert sind. Kationische Tenside adsorbieren an Grenzflächen und aggregieren in wässriger Lösung oberhalb der kritischen Micellbildungskonzentration zu positiv geladenen Micellen.

Beispiele für Kationtenside sind
- quartäre Ammoniumverbindungen, die als hydrophobe Reste ein oder zwei Alkylketten mit einer Kettenlänge von 8 bis 28 C-Atomen tragen können
- quartäre Phosphoniumsalze, substituiert mit einer oder mehreren Alkylketten mit einer Kettenlänge von 8 bis 28 C-Atomen oder
- tertiäre Sulfonium-Salze.

Weiterhin kann die kationische Ladung auch in Form einer Onium-Struktur Bestandteil eines heterozyklischen Ringes (z.B. eines Imidazoliumringe oder einer Pyridiniumrlnges) sein.

Neben der funktionellen Einheit, welche die kationische Ladung trägt, kann das Kationtensid auch weitere ungeladene funktionelle Gruppen beinhalten, diese ist beispielsweise bei Esterquats der Fall. Geeignete kationische Tenside dieses Typs sind beispielsweise physiologisch verträgliche Salze des N,N,N-Trimethyl-1-hexadecanaminiums, insbesondere N,N,N-Trimethyl-1-hexadecanaminiumchlorid, welches auch unter dem Handelsnamen Dehyquart A-CA vertrieben wird.

Bei einem weiteren geeigneten kationischen Tensid handelt es sich um ein physiologisch verträgliches Salze des Dimethyl-distearyldimethylammoniums, besonders bevorzugt um Dimethyldistearylammoniumchlorid.

Weitere kationische Tenside können aus der Gruppe der kationischen Imidazoliumverbindungen ausgewählt werden.

Das erfindungsgemäße Mittel kann das bzw. die kationischen Tenside in einer Gesamtmenge von 0,1 bis 4,8 Gew.-%, bevorzugt von 0,2 bis 2,4 Gew.-%, weiter bevorzugt von 0,3 bis 1,8 Gew.-%- bezogen auf das Gesamtgewicht des Mittels - enthalten.

Weiterhin hat es sich als vorteilhaft erwiesen, wenn die Mittel weitere, nichtionogene grenzflächenaktive Stoffe, enthalten. Bevorzugte nichtionische Tenside sind Alkylpolyglykoside sowie Alkylenoxid-Anlagerungsprodukte an Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Die nichtionischen Tenside werden in Mengen von 0,1 bis 45 Gew.%, bevorzugt 1 bis 30 Gew.% und ganz besonders bevorzugt von 1 bis 15 Gew.%, bezogen auf die Gesamtmenge der Färbemittel, eingesetzt.

In einer weiteren bevorzugten Ausführungsform enthalten die erfindungsgemäßen Mittel neben der Verbindung der Formel (I) zusätzlich mindestens einen weiteren direktziehenden Farbstoff. Durch die Kombination mit weiteren kationischen direktziehenden Farbstoffen lässt sich das erzielbare Nuancenspektrum erweitern, und die Färbeeigenschaften lassen sich noch weiter verbessern.

Direktziehende Farbstoffe können in anionische, kationische und nichtionische direktziehende Farbstoffe unterteilt werden.

Die direktziehenden Farbstoffe sind vorzugsweise ausgewählt aus den kationischen direktziehenden Farbstoffen, da diese mit den Farbstoffen der allgemeinen Formel (I) sehr gut kompatibel sind.

In einer weiteren besonders bevorzugten Ausführungsform ist ein erfindungsgemäßes Mittel dadurch gekennzeichnet, dass es zusätzlich mindestens einen weiteren kationischen direktziehenden Farbstoff enthält, der von den Farbstoffen der Formel (I) verschieden ist.

Als besonders gut kompatibel haben sich ein oder mehrere Farbstoffe aus der Gruppe Basic Yellow 87, Basic Orange 31, Basic Red 51, Basic Violet 2 und Cationic Blue 347 erwiesen.

Ganz besonders gut kompatibel sind die Farbstoffe der Formel (I) mit den kationischen Azofarbstoffen Basic Orange 31 und Basic Red 51. Durch Kombination eines Farbstoffes der Formel (I) mit Basic Orange 31 und/oder Basice Red 51 können - außer rein gelben Nuancen - Nuancen im gesamten Farbspektrum erzeugt werden.

In einer weiteren besonders bevorzugten Ausführungsform ist ein erfindungsgemäßes Mittel dadurch gekennzeichnet, dass es zusätzlich Basic Orange 31 und/oder Basic Red 51 enthält.

Das erfindungsgemäße Mittel kann aber auch zusätzlich mindestens einen nichtionischen direktziehenden Farbstoff enthalten. Dieser kann ausgewählt sein aus der Gruppe HC Yellow 2, HC Yellow 4, HC Yellow 5, HC Yellow 6, HC Yellow 12, HC Orange 1, Disperse Orange 3, HC Red 1, HC Red 3, HC Red 7,HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)aminolbenzoesäure, 4-[(3-Hydroxypropyl)amino]-3-nitrophenol, 4-Nitro-o-phenylendiamin, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Zusätzlich können auch anionische direktziehende Farbstoffe enthalten sein, die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1. Acid Blue 7, Acid Green 50, Acid Violet 43, Acid Black 1, Acid Black 52, Bromphenolblau und Tetrabromphenolblau bekannten sind. Die erfindungsgemäßen Mittel können weiterhin auch zusammen mit Oxidationsfärbemitteln eingesetzt werden. Solche Oxidationsfärbemittel enthalten zusätzlich mindestens ein Oxidationsfarbstoffvorprodukt, bevorzugt zumindest ein Oxidationsfarbstoffvorprodukt vom Entwickler-Typ und mindestens ein Oxidationsfarbstoffvorprodukt vom Kuppler-Typ. Besonders geeignete Oxidationsfarbstoffvorprodukte vom Entwickler-Typ sind dabei ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)pheno|, 4-Amino-2-(diethylaminomethyl)-phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on sowie deren physiologisch verträglichen Salzen.

Besonders geeignete Oxidationsfarbstoffvorprodukte vom Kuppler-Typ sind dabei ausgewählt aus der Gruppe, gebildet aus 3-Aminophenol, 5-Amino-2-methylphenol. 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxy-ethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salzen.

Die zusätzlichen direktziehenden Farbstoffe, Entwicklerkomponenten und Kupplerkomponenten werden bevorzugt jeweils in einem Anteil von 0,0001 bis 5,0 Gew.-%. vorzugsweise 0,001 bis 3,5 Gew.-%, jeweils bezogen auf das anwendungsbereite Mittel, verwendet. Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1 zu 0,5 bis 1 zu 3, insbesondere 1 zu 1 bis 1 zu 2, stehen können.

Soll die Färbung mit den erfindungsgemäßen direktziehenden Farbstoffen der Formel (I) und eine oxidative Aufhellung der Keratinfasern in einem Schritt erfolgen, so enthalten die erfindungsgemäßen Mittel zusätzlich ein Oxidationsmittel, bevorzugt Wasserstoffperoxid und/oder eines seiner festen Anlagerungsprodukte an organische oder anorganische Verbindungen.

In einer bevorzugten Ausführungsform wird Wasserstoffperoxid selbst als wässrige Lösung verwendet. Die Konzentration einer Wasserstoffperoxid-Lösung im erfindungsgemäßen Mittel wird einerseits von den gesetzlichen Vorgaben und andererseits von dem gewünschten Effekt bestimmt; vorzugsweise werden 6 bis 12 Gew.-%ige Lösungen in Wasser verwendet. Erfindungsgemäß bevorzugte anwendungsbereite Mittel des ersten Erfindungsgegenstands sind dadurch gekennzeichnet, dass sie, bezogen auf das Gesamtgewicht des anwendungsbereiten Mittels, 0,5 bis 20 Gew.-%, vorzugsweise 1 bis 12,5 Gew.-%, besonders bevorzugt 2,5 bis 10 Gew.-% und insbesondere 3 bis 6 Gew.-% Wasserstoffperoxid, jeweils bezogen auf das Gesamtgewicht des Mittels, enthalten.

In einer weiteren besonders bevorzugten Ausführungsform ist ein erfindungsgemäßes Mittel dadurch gekennzeichnet, dass es-bezogen auf das Gesamtgewicht des Mittels - 0,5 bis 12,5 Gew.-%, bevorzugt 2,5 bis 10 Gew.-% und insbesondere 3 bis 6 Gew.-% Wasserstoffperoxid enthält.

Zur Erzielung einer verstärkten Aufhell- und Bleichwirkung kann das Mittel weiterhin mindestens ein Peroxo-Salz enthalten. Geeignete Peroxo-Salze sind anorganische Peroxoverbindungen, bevorzugt ausgewählt aus der Gruppe, gebildet aus Ammoniumperoxodisulfat, Alkalimetallperoxodisulfaten, Ammoniumperoxomonosulfat, Alkalimetallperoxomonosulfaten, Alkalimetallperoxodiphosphaten und Erdalkalimetallperoxiden. Besonders bevorzuge sind Peroxodisulfate, insbesondere Ammoniumperoxodisulfat, Kaliumperoxodisulfat und Natriumperoxodisulfat.

In einer weiteren besonders bevorzugten Ausführungsform ist ein erfindungsgemäßes Mittel dadurch gekennzeichnet, dass es zusätzlich mindestens ein Persulfat aus der Gruppe Ammoniumperoxodisulfat, Kaliumperoxodisulfat und Natriumperoxodisulfat enthält.

Die Persulfate sind jeweils in einer Menge von 0,5 bis 20 Gew.-%, vorzugsweise 1 bis 12,5 Gew.-%, besonders bevorzugt 2,5 bis 10 Gew.-% und insbesondere 3 bis 6 Gew.-%, bezogen auf das Gesamtgewicht des anwendungsbereiten Mittels, in dem erfindungsgemäßen Mittel enthalten.

Das Färbe- und/oder Mattier-Mittel kann zur Verstärkung der Blondierwirkung weitere Bleichkraftverstärker enthalten, wie beispielsweise Tetraacetylethylendiamin (TAED), 1,5-Diacetyl-2,4-dioxo-hexahydro-1,3,5-triazin (DADHT), Tetraacetylglykoluril (TAGU), N-Nonanoylsuccinimid (NOSI), n-Nonanoyl- oder Isononanoyloxybenzolsulfonat (n- bzw. i-NOBS), Phthalsäureanhydrid, Triacetin, Ethylenglykoldiacetat und 2,5-Diacetoxy-2,5-dihydrofuran sowie Carbonatsalze bzw. Hydrogencarbonatsalze, insbesondere Ammoniumhydrogencarbonat, Ammoniumcarbonat, Natriumhydrogencarbonat, Dinatriumcarbonat, Kaliumhydrogencarbonat, Dikaliumcarbonat und Calciumcarbonat, und stickstoffhaltige, heterocyclische Bleichkraftverstärker, wie 4-Acetyl-1-methylpyridinium-p-toluolsulfonat, 2-Acetyl-1-methylpyridinium-p-toluolsulfonat, sowie N-Methyl-3,4-dihydroisochinolinium-p-toluolsulfonat.

Zur weiteren Steigerung der Aufhellung kann der erfindungsgemäßen Zusammensetzung zusätzlich mindestens eine SiO₂-Verbindung, wie Kieselsäure oder Silicate, insbesondere Wassergläser, zugesetzt sein. Es kann erfindungsgemäß bevorzugt sein, die SiO₂-Verbindungen in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.-% bis 5 Gew.-%, Jeweils bezogen auf die erfindungsgemäße wasserfreie Zusammensetzung, einzusetzen. Die Mengenangaben geben dabei jeweils den Gehalt der SiO₂-Verbindungen (ohne deren Wasseranteil) in den Mitteln wieder.

Die Färbemittel können weiterhin zusätzliche Wirk-, Hilfs- und Zusatzstoffe enthalten, um die Färbeleistung zu verbessern und weitere gewünschte Eigenschaften der Mittel einzustellen.

Vorzugsweise werden die Färbemittel als flüssige Zubereitung bereitgestellt und den Mitteln daher gegebenenfalls zusätzlich eine weitere oberflächenaktive Substanz zugesetzt, wobei solche oberflächenaktive Substanzen je nach Anwendungsgebiet als Tenside oder als Emulgatoren bezeichnet werden: Sie sind bevorzugt aus anionischen, zwitterionischen, amphoteren und nichtionischen Tensiden und Emulgatoren ausgewählt.

Die erfindungsgemäßen Färbemittel können weitere Hilfs- und Zusatzstoffe enthalten. So hat es sich als vorteilhaft erwiesen, wenn die Mittel mindestens ein Verdickungsmittel enthält. Bezüglich dieser Verdickungsmittel bestehen keine prinzipiellen Einschränkungen. Es können sowohl organische als auch rein anorganische Verdickungsmittel zum Einsatz kommen.

Geeignete Verdickungsmittel sind
- anionische, synthetische Polymere;
- kationische, synthetische Polymere:
- natürlich vorkommende Verdickungsmittel, wie nichtlonische Guargums, Skleroglucangums oder Xanthangums, Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, sowie Cellulosederivate, wie beispielsweise Methylcellulose, Carboxyalkylcellulosen und Hydroxyalkylcellulosen;
- nichtionische, vollsynthetische Polymere, wie Polyvinylalkohol oder Polyvinylpyrrolidinon; sowie
- anorganische Verdickungsmittel, insbesondere Schichtsilikate wie beispielsweise Bentonit, besonders Smektite, wie Montmorillonit oder Hectorit.

Färbeprozesse auf Keratinfasern laufen üblicherweise im alkalischen Milieu ab. Um die Keratinfasern und auch die Haut so weit wie möglich zu schonen, ist die Einstellung eines zu hohen pH-Wertes jedoch nicht wünschenswert. Daher ist es bevorzugt, wenn der pH-Wert des anwendungsbereiten Mittels zwischen 7 und 11, insbesondere zwischen 8 und 10,5, liegt. Bei den pH-Werten im Sinne der vorliegenden Erfindung handelt es sich um pH-Werte, die bei einer Temperatur von 22 °C gemessen wurden.

Die zur Einstellung des bevorzugten pH-Wertes erfindungsgemäß verwendbaren Alkalisierungsmittel können aus der Gruppe, die gebildet wird aus Ammoniak, Alkanolaminen, basischen Aminosäuren, sowie anorganischen Alkalisierungsmitteln wie (Erd-)Alkalimetallhydroxiden, (Erd-)-Alkalimetallmetasilikaten, (Erd-)Alkalimetallphosphaten und (Erd-)Alkalimetallhydrogenphosphaten, ausgewählt werden. Bevorzugte anorganische Alkalisierungsmittel sind Magnesiumcarbonat, Natriumhydroxid, Kaliumhydroxid, Natriumsilicat und Natriummetasilicat. Erfindungsgemäß einsetzbare, organische Alkalisierungsmittel werden bevorzugt ausgewählt aus Monoethanolamin, 2-Amino-2-methylpropanol und Triethanolamin. Die als erfindungsgemäßes Alkalisierungsmittel einsetzbaren basischen Aminosäuren werden bevorzugt ausgewählt aus der Gruppe, die gebildet wird aus Arginin, Lysin, Ornithin und Histidin, besonders bevorzugt Arginin. Es hat sich aber im Rahmen der Untersuchungen zur vorliegenden Erfindung herausgestellt, dass weiterhin erfindungsgemäß bevorzugte Mittel dadurch gekennzeichnet sind, dass sie zusätzlich ein organisches Alkalisierungsmittel enthalten. Eine Ausführungsform des ersten Erfindungsgegenstands ist dadurch gekennzeichnet, dass das Mittel zusätzlich mindestens ein Alkalisierungsmittel enthält, welches ausgewählt ist aus der Gruppe, die gebildet wird aus Ammoniak, Alkanolaminen und basischen Aminosäuren, insbesondere aus Ammonlak, Monoethanolamin und Arginin oder seinen verträglichen Salzen.

Weiterhin hat es sich als vorteilhaft erweisen, wenn die Färbemittel, insbesondere wenn sie zusätzlich Wasserstoffperoxid enthalten, mindestens einen Stabilisator oder Komplexbildner enthalten. Besonders bevorzugte Stabilisatoren sind Phenacetin, Alkalibenzoate (Natriumbenzoat) und Salicylsäure. Weiterhin können alle Komplexbildner des Standes der Technik eingesetzt werden. Erfindungsgemäß bevorzugte Komplexbildner sind stickstoffhaltigen Polycarbonsäuren, insbesondere EDTA und EDDS, und Phosphonate, insbesondere 1-Hydroxyethan-1,1-diphosphonat (HEDP) und/oder Ethylendiamintetramethylenphosphonat (EDTMP) und/oder Diethylentriaminpentamethylenphosphonat (DTPMP) bzw. deren Natriumsalze.

Ferner können die erfindungsgemäßen Mittel weitere Wirk-, Hilfs- und Zusatzstoffe enthalten, wie beispielsweise nichtionische Polymere wie beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane; zusätzliche Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Polyoxyalkylen(B)-Blockcopolymere, gepfropften Silikonpolymere; kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylamino-ethylmethacrylat-Vinylpyrrolidinon-Copolymere, Vinylpyrrolidinon-Imidazolinium-methochlorid-Copolymere und quaternierter Polyvinylalkohol; zwitterionische und amphotere Polymere; anionische Polymere wie beispielsweise Polyacrylsäuren oder vernetzte Polyacrylsäuren; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und - distearat sowie PEG-3-distearat; Pigmente sowie Treibmittel wie Propan-Butan-Gemische, N₂O, Dimethylether, CO₂ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen.

Die zusätzlichen Wirk- und Hilfsstoffe werden in den erfindungsgemäßen Mitteln bevorzugt in Mengen von jeweils 0,0001 bis 25 Gew.-%, insbesondere von 0,0005 bis 15 Gew.-%, bezogen auf das Gesamtgewicht der Anwendungsmischung, eingesetzt.

Mit den Färbemitteln, welche die erfindungsgemäßen direktziehenden Farbstoffe der allgemeinen Formel (I) enthalten, lassen sich keratinische Fasern in ausgesprochen attraktiven und intensiven Blautönen färben. Bei den Farbstoffen der allgemeinen Formel (I) handelt es sich um kationische dimere Azofarbstoffe. Hierbei hat sich überraschenderweise herausgestellt, dass die Farbintensität von kationischen Azofarbstoffen durch Zusatz eines oder mehrere anionischer Tenside (b) der Formel (B1) noch weiter gesteigert werden kann,

R-O-(CH₂-CH₂O)x-CH2-COOM (B1),

wobei
- R: für eine lineare Alkylgruppe mit 8 bis 30 C-Atomen steht,
- x: für eine ganze Zahl von 0 bis 16 steht,
- M: für Wasserstoff oder ein Metall wie ein Alkalimetall, insbesondere Natrium, Kalium, Lithium, ein Erdalkalimetall, insbesondere ½ Magnesium, ½ Calcium, ½ Zink, oder ein Ammoniumion (NH4⁺) steht.

Unter der Verbesserung des Farbaufzugsvermögens wird in diesem Zusammenhang verstanden, dass die Farbstoffe vermehrt bzw. verstärkt in die keratinische Faser hineindiffundieren, was zu Färbungen mit höherer Farbintensität führt. Die verstärkte Farbintensität lässt sich enweder visuell durch Betrachtung unter einer Tageslichtlampe oder aber durch farbmetrische Vermessung (Bestimmung der Lab-Werte) detektieren.

Geeigente kationische Azofarbstoffe sind beispielsweise die Farbstoffe Basic Yellow, Basic Orange 31 und Basic Red 51.

Die Intensivierung des Farbaufzugs durch die anionischen Tenside der Formel (B1) funktioniert zwar prinzipiell bei allen kationischen Azofarbstoffen. Besonders gut lässt sich durch Zugabe der anionischen Tenside der Formel (B1) jedoch der Farbaufzug der Farbstoffe der allgemeinen Formel (I) verbessern.

Die Mittel des ersten Erfindungsgegenstands können in Verfahren zum Färben und auch in Verfahren zum gleichzeitigen Blondieren bzw. Aufhellen und Färben menschlicher Haare genutzt werden.

Die erfindungsgemäßen Mittel können als Einkomponentenmittel oder als Mehrkomponentenmittel wie Zweikomponenten Mittel oder Dreikomponentenmittel formuliert und entsprechend angewendet werden. Eine Auftrennung in Mehrkomponentensysteme bietet sich insbesondere dort an, wo Inkompatibilitäten der Inhaltsstoffe zu erwarten oder zu befürchten sind; das einzusetzende Mittel wird bei solchen Systemen vom Verbraucher direkt vor der Anwendung durch Vermischen der Komponente hergestellt.

Unter dem erfindungsgemäßen Mittel zur Farbveränderung von keratinischen Fasern wird immer das anwendungsbereite Mittel verstanden.

Wird das erfindungsgemäße Mittel dem Anwender in Form eines Einkomponenten-Mittels zur Verfügung gestellt, dann muss das anwendungsbereite Mittel nicht erst hergestellt werden, sondern kann direkt aus dem Behältnis, in dem es konfektioniert wurde, entnommen und auf die keratinischen Fasern appliziert werden.

Bei Blondiermitteln handelt es sich jedoch üblicherweise um Zweikomponentenprodukte, bei welchen eine oxidationsmittelhaltige Komponente (A1) kurz vor der Anwendung mit einem (Alkalisierungs-)Mittel (A2) vermischt und diese anwendungsbereite Mischung auf die Haare aufgetragen wird.

In diesem Fall handelt es sich bei dem erfindungsgemäßen Mittel um das anwendungsbereite Mittel, welches kurz vor der Anwendung durch Vermischen von (A1) und (A2) hergestellt wurde.

Hierbei können die direktziehenden Farbstoffe der allgemeinen Formel (I) in der Komponente (A1) (d.h. zusammen mit dem Oxidationsmittel) oder aber in der Komponente (A2) (zusammen mit dem Alkalisierungsmittel) konfektioniert werden.

Es ist ebenfalls möglich und erfindungsgemäß, wenn das anwendungsbereite Mittel kurz vor der Anwendung auf den menschlichen Haaren durch Vermischen von 3 Komponenten hergestellt wird, wobei
- die Komponente (A1) mindestens einen direktziehenden Farbstoff der allgemeinen Formel (I) und mindestens ein Alkalisierungsmittel
- die Komponente (A2) mindestens ein erstes Oxidationsmittel (z.B. Wasserstoffperoxid) und
- die Komponente (A3) mindestens ein zweites Oxidationsmittel (z.B. ein oder mehrere Poroxodisulfatsalze) enthält.

Während der Einwirkzeit der Mittel auf der Faser kann es vorteilhaft sein, den Aufhellvorgang oder Mattiervorgang durch Wärmezufuhr zu unterstützen. Die Wärmezufuhr kann durch eine externe Wärmequelle, wie z.B. warme Luft eines Warmluftgebläses, als auch, insbesondere bei einer Haaraufhellung am lebenden Probanden, durch die Körpertemperatur des Probanden erfolgen. Bei letzterer Möglichkeit wird üblicherweise die behandelte Partie mit einer Haube abgedeckt. Eine Einwirkphase bei Raumtemperatur ist ebenfalls erfindungsgemäß. Insbesondere liegt die Temperatur während der Einwirkzeit zwischen 20 °C und 40 °C, insbesondere zwischen 25 °C und 38 °C. Nach Ende der Einwirkzeit wird die verbleibende Färbezubereitung mit Wasser oder einem Reinigungsmittel aus dem Haar gespült. Als Reinigungsmittel kann dabei insbesondere handelsübliches Shampoo dienen, wobei insbesondere dann auf das Reinigungsmittel verzichtet werden kann und der Ausspülvorgang mit Wasser erfolgen kann, wenn das Aufhellmittel einen stark tensidhaltigen Träger besitzt.

Bezüglich weiterer bevorzugter Ausführungsformen der erfindungsgemäßen Verwendungen gilt mutatis mutandis das zu den erfindungsgemäßen Mitteln Gesagte.

### Beispiele

### Direkzieher 1: 3-Methyl-2-(2-{4-[methyl({3-[methyl({4-[2-(3-methyl-1,3-thiazol-3-ium-2-yl)diazen-1-yl]phenyl})amino]propyl})amino]phenyl}diazen-1-yl)-1,3-thiazol-3-ium, Di(methylsulfat) (DZ 1, erfindungsgemäß)

Der Farbstoff DZ 1 wurde nach einem Verfahren synthetisiert, wie es in den Dokumenten WO 2002/100369 A2 und US 3291788 beschrieben ist.

Als Edukte wurden 2-Aminothiazol und N,N'-Dimethyl-N,N'-diphenyl-propan-1,3-diamin eingesetzt (Azokupplungsreaktion in wässriger, schwefelsauerer Lösung mit Nitrosylschwefelsäure). Der in dieser Azokupplungsreaktion entstehende neutrale Farbstoff wurde im Anschluss daran quaterniert (beispielsweise mit dem Quaternierungsmittel Dimethylsulfat in einem polaren, aprotischen Lösungsmittel wie Dimethylformamid oder Dimethylsulfoxid).

### DZ 1 (erfindungsgemäß)

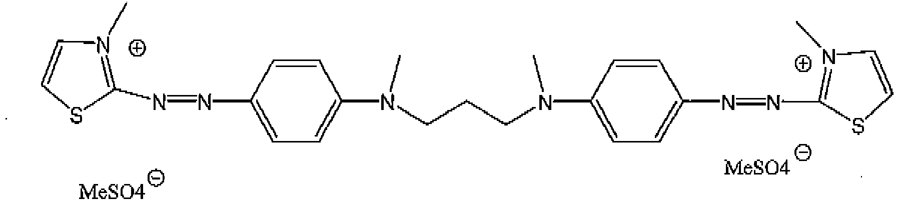

### Vergleichsbeispiel

### Direkzieher 2: 3-Methyl-2-(2-{4-[methyl({2-[methyl({4-[2-(3-methyl-1,3-thiazol-3-ium-2-yl)diazen-1-yl]phenyl})amino]ethyl})amino]phenyl}diazen-1-yl)-1,3-thiazol-3-ium Di(methylsulfat) (DZ 2, Vergleich)

Der Farbstoff DZ 2 wurde nach einem Verfahren synthetisiert, wie es in den Dokumenten WO 2002/100369 A2 und US 3291788 beschrieben ist.

Als Edukte wurden 2-Aminothiazol und N,N'-Dimethyl-N,N'-diphenyl-ethan-1,2-diamin eingesetzt (Azokupplungsreaktion in wässriger, schwefelsauerer Lösung mit Nitrosylschwefelsäure). Der in dieser Azokupplungsreaktion entstehende Farbstoff wurde im Anschluss daran quaterniert (beispielsweise mit einem Quaternierungsmittel wie Dimethylsulfat in einem polaren, aprotischen Lösungsmittel wie Dimethylformamid oder Dimethylsulfoxid).

### DZ 2 (Vergleich):

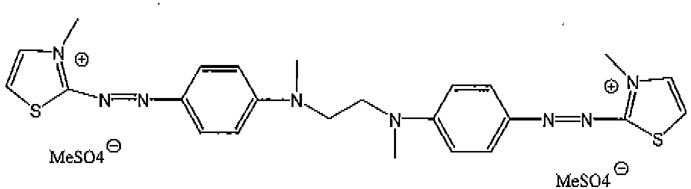

### Färbebeispiele

### Formulierungen

Es wurden die folgenden Färbecremes hergestellt (alle Angaben in Gew.-%, Aktivsubstanz)

| | **V1** | **V2** | **E1** | **E2** |
|---|---|---|---|---|
| Cetearyl Alcohol (C16/C18-Fettalkohol) | 1,0 | 1,0 | 1,0 | 1,0 |
| Coconut Alcohol (C12/C18-Fettalkohol) | 1,0 | 1,0 | 1,0 | 1,0 |
| Methylparaben | 0,1 | 0,1 | 0,1 | 0,1 |
| Propylparaben | 0,1 | 0,1 | 0,1 | 0,1 |
| Natrium Laureth-5 Carboxylat | --- | 1,0 | --- | 1,0 |
| Natrium Laureth Sulfat (2 EO) | 1,0 | --- | 1,0 | --- |
| DZ 1 (erfindungsgemäß) | --- | --- | 1,0 | 1,0 |
| DZ 2 (Vergleich) | 1,0 | 1,0 | --- | --- |
| Ammoniumsulfat | 1,0 | 1,0 | 1,0 | 1,0 |
| Wasser | ad 100 | ad 100 | ad 100 | ad 100 |

Die ersten fünf Komponenten wurden zusammen aufgeschmolzen. Diese Schmelze wurde mit heißem Wasser emulgiert, anschließend wurde der in Propylenglycol vorgelöste Farbstoff hinzu gegeben sowie die Ammoniumsulfatlösung hinzugefügt. Der angegebene pH-Wert wurde mit Ammoniak eingestellt, danach wurde mit Wasser auf 100 g aufgefüllt.

### Ausfärbungen

Jeweils 1,8 g der Färbecreme wurden auf eine ca. 6 cm lange Strähne Menschenhaares (Kerling Euronaturhaar,80 % ergraut) aufgetragen und dort 30 Minuten bei 30 °C belassen. Nach Beendigung der Einwirkzeit wurde das Haar ausgespült, mit einem üblichen Haarwaschmittel gewaschen und anschließend getrocknet. Nach dem Trocknen wurden die Färbung und die Farbintensität der Strähnen visuell unter der Tageslichtlampe beurteilt.

| | Formulierung | pH-Wert | Farbnuance | Farbintensität |
|---|---|---|---|---|
| V1 | DZ 2 (Vergleich) mit Natrium Laureth Sulfat (2 EO) | 9,5 | grau (Ausgangshaarfarbe, kein Farbaufzug) | + |
| V2 | DZ 2 (Vergleich) mit Natrium Laureth-5 Carboxylat | 9,5 | grau (Ausgangshaarfarbe, kein Farbaufzug) | + |
| E1 | DZ 1 (erfindungsgemäß) mit Natrium Laureth Sulfat (2 EO) | 9,5 | tiefblau | ++++ |
| E2 | DZ 1 (erfindungsgemäß) mit Natrium Laureth-5 Carboxylat | 9,5 | schwarzblau | +++++ |

| | | | | |
|---|---|---|---|---|
| Farbintensität: + = schlecht +++ = mittel +++++ = sehr gut | | | | |

Bei den Formulierungen V1 und V2 handelt es sich um Vergleichsformulierungen, welche den nicht erfindungsgemäßen direktziehenden Farbstoff DZ 2 enthielten (DZ 2: 3-Methyl-2-(2-{4-[methyl({2-[methyl({4-[2-(3-methyl-1,3-thiazol-3-ium-2-yl)diazen-1-yl]phenyl})amino]ethyl})amino]phenyl}diazen-1-yl)-1,3-thiazol-3-ium Di(methylsulfat) (Vergleich)).

Bei Ausfärbung von V1 und V2 konnte kein Farbaufzug beobachtet werden, die Strähnen waren wie die Ausgangshaarfarbe (Kerling, zu 80 % ergraut) grau gefärbt.

Bei den Formulierungen E1 und E2 handelt es sich um erfindungsgemäße Formulierungen.

Bei Kombination des Farbstoffes DZ 1 mit dem anionischen Tensid Natrium Laureth Sulfat (2 EO) wurde eine tiefblaue Färbung mit hoher Farbintensität beobachtet (E1).

Bei Kombination des Farbstoffes DZ 1 mit dem anionischen Tensid Natrium Laureth-5 Carboxylat konnte eine noch intensivere, schwarzblaue Färbung erzielt werden.

## Patentansprüche

1. Mittel zum Färben von keratinischen Fasern, insbesondere menschlichen Haaren, enthaltend in einem kosmetischen Träger
(a) mindestens einen direktziehenden Farbstoff der Formel (I), 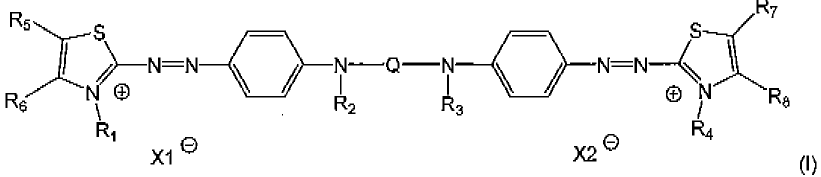 wobei
R1, R4 unabhängig voneinander für eine C₁-C₈-Alkylgruppe stehen,
R2, R3 unabhängig voneinander für ein Wasserstoffatom oder für eine C₁-C₈-Alkylgruppe stehen,
R5, R6, R7, R8 jeweils für ein Wasserstoffatom stehen.
X1, X2 unabhängig voneinander für ein physiologisch verträgliches Anion, bevorzugt aus der Gruppe Chlorid, Bromid, lodid, Methylsulfat, Methylsulfonat, p-Toluolsulfonat, Acetat, Hydrogensulfat, ½ Sulfat oder ½ Tetrachlorozinkat stehen,
Q für eine Gruppierung der Formel (II) steht,
*-(CH₂)n-* (II)
n für eine ganze Zahl von 3 bis 6 steht,
und
(b) mindestens ein anionisches Tensid.

2. Mittel nach Anspruch 1, **dadurch gekennzeichnet, dass** es (a) mindestens einen direktziehenden Farbstoff der allgemeinen Formel (I) enthält, der ausgewählt ist aus
- Salzen des 3-Methyl-2-[2-(4-{[3-({4-[2-(3-methyl-1,3-thiazol-3-ium-2-yl)diazen-1-yl]phenyl}amino)propyl]amino}phenyl)diazen-1-yl]-1,3-thiazol-3-iums
- Salzen des 3-Methyl-2-[2-(4-{[4-({4-[2-(3-methyl-1,3-thiazol-3-ium-2-yl)diazen-1-yl]phenyl}amino)butyl]amino}phenyl)diazen-1-yl]-1,3-thiazol-3-iums
- Salzen des 3-Methyl-2-[2-(4-{[5-({4-[2-(3-methyl-1,3-thiazol-3-ium-2-yl)diazen-1-yl]phenyl}amino)pentyl]amino}phenyl)diazen-1-yl]-1,3-thiazol-3-iums
- Salzen des 3-Methyl-2-(2-{4-[methyl({3-[methyl({4-[2-(3-methyl-1,3-thiazol-3-ium-2-yl)diazen-1-yl]phenyl})amino]propyl})amino]phenyl}diazen-1-yl)-1,3-thiazol-3-iums
- Salzen des 3-Methyl-2-(2-{4-[methyl({4-[methyl({4-[2-(3-methyl-1,3-thiazol-3-ium-2-yl)diazen-1-yl]phenyl})amino]butyl})amino]phenyl}diazen-1-yl)-1,3-thiazol-3-iums
- Salzen des 3-Methyl-2-(2-{4-[methyl({5-[methyl({4-[2-(3-methyl-1,3-thiazol-3-ium-2-yl)diazen-1-yl]phenyl})amino]pentyl})amino]phenyl}diazen-1-yl)-1,3-thiazol-3-iums
- Salzen des 2-(2-{4-[Ethyl({3-[ethyl({4-[2-(3-methyl-1,3-thiazol-3-ium-2-yl)diazen-1-yl]phenyl})amino]propyl})amino]phenyl}diazen-1-yl)-3-methyl-1,3-thiazol-3-iums
- Salzen des 2-(2-{4-[Ethyl({4-[ethyl({4-[2-(3-methyl-1,3-thiazol-3-ium-2-yl)diazen-1-yl]phenyl})amino]butyl})amino]phenyl}diazen-1-yl)-3-methyl-1,3-thiazol-3-iums
- Salzen des 2-(2-{4-[Ethyl({5-[ethyl({4-[2-(3-methyl-1,3-thiazol-3-ium-2-yl)diazen-1-yl]phenyl})amino]pentyl})amino]phenyl}diazen-1-yl)-3-methyl-1,3-thiazol-3-iums
- Salzen des 3-Ethyl-2-[2-(4-{[3-({4-[2-(3-ethyl-1,3-thiazol-3-ium-2-yl)diazen-1-yl]phenyl}amino)propyl]amino}phenyl)diazen-1-yl]-1,3-thiazol-3-iums
- Salzen des 3-Ethyl-2-[2-(4-{[4-({4-[2-(3-ethyl-1,3-thiazol-3-ium-2-yl)diazen-1-yl]phenyl}amino)butyl]amino}phenyl)diazen-1-yl]-1,3-thiazol-3-iums
- Salzen des 3-Ethyl-2-[2-(4-{[5-({4-[2-(3-ethyl-1,3-thiazol-3-ium-2-yl)diazen-1-yl]phenyl}amino)pentyl]amino}phenyl)diazen-1-yl]-1,3-thiazol-3-iums
- Salzen des 3-Ethyl-2-[2-(4-{[3-({4-[2-(3-ethyl-1,3-thiazol-3-ium-2-yl)diazen-1-yl]phenyl}(methyl)amino)propyl](methyl)amino}phenyl)diazen-1-yl]-1,3-thiazol-3-iums
- Salzen des 3-ethyl-2-[2-(4-{[4-({4-[2-(3-ethyl-1,3-thiazol-3-ium-2-yl)diazen-1-yl]phenyl}(methyl)amino)butyl](methyl)amino}phenyl)diazen-1-yl]-1,3-thiazol-3-iums
- Salzen des 3-Ethyl-2-[2-(4-{[5-({4-[2-(3-ethyl-1,3-thiazol-3-ium-2-yl)diazen-1-yl]phenyl}(methyl)amino)pentyl](methyl)amino}phenyl)diazen-1-yl]-1,3-thiazol-3-iums
- Salzen des 3-Ethyl-2-(2-{4-[ethyl({3-[ethyl({4-[2-(3-ethyl-1,3-thiazol-3-ium-2-yl)diazen-1-yl]phenyl})amino]propyl})amino]phenyl}diazen-1-yl)-1,3-thiazol-3
- Salzen des 3-Ethyl-2-(2-{4-[ethyl({4-[ethyl({4-[2-(3-ethyl-1,3-thiazol-3-ium-2-yl)diazen-1-yl]phenyl})amino]butyl})amino]phenyl}diazen-1-yl)-1,3-thiazol-3-iums und/oder
- Salzen des 3-Ethyl-2-(2-{4-[ethyl({5-[ethyl({4-[2-(3-ethyl-1,3-thiazol-3-ium-2-yl)diazen-1-yl]phenyl})amino]pentyl})amino]phenyl}diazen-1-yl)-1,3-thiazol-3-iums.

3. Mittel nach einem der Ansprüche 1 bis 2, **dadurch gekennzeichnet, dass** es - bezogen auf das Gesamtgewicht des Mittels - einen oder mehrere direktziehende Farbstoffe (a) der Formel (I) in einer Gesamtmenge von 0,01 bis 4,5 Gew.-%, bevorzugt von 0.05 bis 2,8 Gew.-%, weiter bevorzugt von 0,1 bis 2,2 Gew.-% und besonders bevorzugt von 0,2 bis 1,2 Gew.-% enthält.

4. Mittel nach einem der Ansprüche 1 bis 3, **dadurch gekennzeichnet, dass** es (b) mindestens ein anionisches Tensid enthält, das ausgewählt ist aus den
- linearen und verzweigten Fettsäuren mit 8 bis 30 C-Atomen,
- Ethercarbonsäuren der Formel R-O-(CH₂-CH₂O)ₓ-CH₂-COOM, in der
R für eine lineare Alkylgruppe mit 8 bis 30 C-Atomen steht,
X für eine ganze Zahl von 0 bis 16 steht,
M für Wasserstoff oder ein Metall wie Alkalimetall, insbesondere Natrium, Kalium, Lithium, Erdalkalimetall, insbesondere Magnesium, Calcium, Zink, oder ein Ammoniumion (NH4⁺) steht,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe, die durch Veresterung von C₈-C₂₄-Fettsäuren mit dem Natriumsalz der 2-Hydroxyethan-sulfonsäure (Isethionsäure) zugänglich sind,
- Amidethercarbonsäuren, R¹-CO-NR²-CH₂CH₂-O-(CH₂CH₂O)_{y}CH₂COOM, wobei
R¹ für eine C2-C30-Alkylgruppe steht,
y für eine ganze Zahl von 1 bis 20 steht,
R² für Wasserstoff, einen Methyl-, Ethyl-, Propyl-, Isopropyl-, n-Butyl-, t-Butyl- oder iso-Butylrest steht, und
M für Wasserstoff oder ein Metall wie Alkalimetall, insbesondere Natrium, Kalium, Lithium, Erdalkalimetall, insbesondere Magnesium, Calcium, Zink, oder ein Ammoniumion (NH4⁺) steht.

5. Mittel nach einem der Ansprüche 1 bis 4, **dadurch gekennzeichnet, dass** es als anionisches Tensid (b) mindestens eine Ethercarbonsäure der Formel (B1) enthält,
R-O-(CH2-CH2O)x-CH2-COOM (B1),
wobei
R für eine lineare Alkylgruppe mit 8 bis 30 C-Atomen steht,
x für eine ganze Zahl von 0 bis 16 steht,
M für Wasserstoff oder ein Metall wie ein Alkalimetall, insbesondere Natrium, Kalium, Lithium, ein Erdalkalimetall, insbesondere ½ Magnesium, ½ Calcium, ½ Zink, oder ein Ammoniumion (NH4⁺) steht.

6. Mittel nach einem der Ansprüche 1 bis 5, **dadurch gekennzeichnet, dass** es - bezogen auf das Gesamtgewicht des Mittels - ein oder mehrere anionische Tenside (b) in einer Gesamtmenge von 0,05 bis 4,5 Gew.-%. bevorzugt von 0,1 bis 3,1 Gew.-%, weiter bevorzugt von 0,15 bis 2,5 Gew.-% und ganz besonders bevorzugt von 0,2 bis 0,9 Gew.-% enthält.

7. Mittel nach einem der Ansprüche 1 bis 6, **dadurch gekennzeichnet, dass** das Gewichtsverhältnis aus allen im Mittel enthaltenen anionischen Tensiden der Formel (B1) zur Gesamtmenge der im Mittel enthaltenen anionischen Tenside bei einem Wert von 0,5, bevorzugt von 0,6, weiter bevorzugt von 0,75 und besonders bevorzugt von 0,9 liegt.

8. Mittel nach einem der Ansprüche 1 bis 7, **dadurch gekennzeichnet, dass** es zusätzlich mindestens einen weiteren kationischen direktztiehenden Farbstoff enthält, der von den Farbstoffen der Formel (I) verschieden ist.

9. Mittel nach einem der Ansprüche 1 bis 8, **dadurch gekennzeichnet, dass** es zusätzlich Basic Orange 31 und/oder Basic Red 51 enthält,

10. Mittel nach einem der Ansprüche 1 bis 9, **dadurch gekennzeichnet, dass** es - bezogen auf das Gesamtgewicht des Mittels - 0,5 bis 12,5 Gew.-%, bevorzugt 2,5 bis 10 Gew.-% und insbesondere 3 bis 6 Gew.-% Wasserstoffperoxid enthält.

11. Mittel nach einem der Ansprüche 1 bis 10, **dadurch gekennzeichnet, dass** es zusätzlich mindestens ein Persulfat aus der Gruppe Ammoniumperoxodisulfat, Kaliumperoxodisulfat und Natriumperoxodisulfat enthält.

## Claims

1. An agent for dyeing keratin fibers, in particular human hair, containing, in a cosmetic carrier,
(a) at least one substantive dye of formula (I) 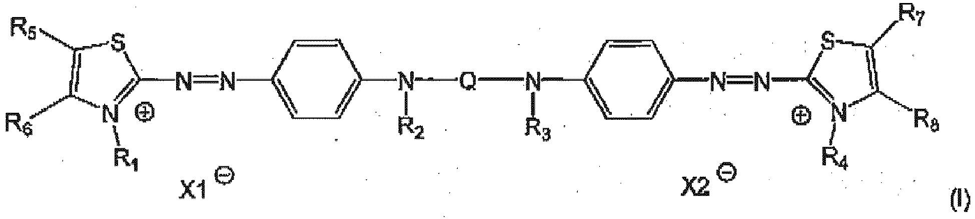 wherein
R1 and R4 represent, independently of one another, a C₁-C₈ alkyl group,
R2 and R3 represent, independently of one another, a hydrogen atom or a C₁-C₈ alkyl group,
R5, R6, R7 and R8 each represent a hydrogen atom,
X1 and X2 represent, independently of one another, a physiologically acceptable anion, preferably from the group chloride, bromide, iodide, methylsulfate, methylsulfonate, p-toluenesulfonate, acetate, hydrogen sulfate, ½ sulfate or ½ tetrachlorozincate,
Q represents a group of formula (II),
*-(CH₂)n-* (II)
n represents an integer from 3 to 6, and
(b) at least one anionic surfactant.

2. The agent according to claim 1, **characterized in that** it contains (a) at least one substantive dye of general formula (I), which is selected from
- salts of 3-methyl-2-[2-(4-{[3-({4-[2-(3-methyl-1,3-thiazol-3-ium-2-yl)diazene-1-yl]phenyl}amino)propyl]amino}phenyl)diazene-1-yl]-1,3-thiazol-3-ium
- salts of 3-methyl-2-[2-(4-{[4-({4-[2-(3-methyl-1-3-thiazol-3-ium-2-yl)diazene-1 yl]phenyl}amino)butyl]amino}phenyl)diazene-1-yl]-1,3-thiazol-3-ium
- salts of 3-methyl-2-[2-(4-{[5-({4-[2-(3-methyl-1,3-thiazol-3-ium-2-yl)diazene-1-yl]phenyl}amino)pentyl]amino}phenyl)diazene-1-yl]-1,3-thiazol-3-ium
- salts of 3-methyl-2-(2-{4-[methyl({3-[methyl({4-[2-(3-methyl-1,3-thiazol-3-ium-2-yl)diazene-1-yl]phenyl})amino]propyl})amino]phenyl}diazene-1-yl)-1,3-thiazol-3-ium
- salts of 3-methyl-2-(2-{4-[methyl({4-[methyl({4-[2-(3-methyl-1,3-thiazol-3-ium-2-yl)diazene-1-yl]phenyl})amino]butyl})amino]phenyl}diazene-1-yl)-1,3-thiazol-3-ium
- salts of 3-methyl-2-(2-{4-[methyl({5-[methyl({4-[2-(3-methyl-1,3-thiazol-3-ium-2-yl)diazene-1-yl]phenyl})amino]pentyl})amino]phenyl)diazene-1-yl)-1,3-thiazol-3-ium
- salts of 2-(2-{4-[ethyl({3-[ethyl({4-[2-(3-methyl-1,3-thiazol-3-ium-2-yl)diazene-1-yl]phenyl})amino]propyl})amino]phenyl}diazene-1-yl)-3-methyl-1,3-thiazol-3-ium
- salts of 2-(2-{4-[ethyl({4-[ethyl({4-[2-(3-methyl-1,3-thiazol-3-ium-2-yl)diazene-1-yl]phenyl})amino]butyl})amino]phenyl}diazene-1-yl)-3-methyl-1,3-thiazol-3-ium
- salts of 2-(2-{4-[ethyl({5-[ethyl({4-[2-(3-methyl-1,3-thiazol-3-ium-2-yl)diazene-1-yl]phenyl})amino]pentyl})amino]phenyl}diazene-1-yl)-3-methyl-1,3-thiazol-3-ium
- salts of 3-ethyl-2-[2-(4-{[3-({4-[2-(3-ethyl-1,3-thiazol-3-ium-2-yl)diazene-1-yl]phenyl}amino)propyl]amino}phenyl)diazene-1-yl]-1,3-thiazol-3-ium
- salts of 3-ethyl-2-[2-(4-{[4-({4-[2-(3-ethyl-1,3-thiazol-3-ium-2-yl)diazene-1-yl]phenyl}amino)butyl]amino}phenyl)diazene-1-yl]-1,3-thiazol-3-ium
- salts of 3-ethyl-2-[2-(4-{[5-({4-[2-(3-ethyl-1,3-thiazol-3-ium-2-yl)diazene-1-yl]phenyl}amino)pentyl]amino}phenyl)diazene-1-yl]-1,3-thiazol-3-ium
- salts of 3-ethyl-2-[2-(4-{[3-({4-[2-(3-ethyl-1,3-thiazol-3-ium-2-yl)diazene-1-yl]phenyl}(methyl)amino)propyl](methyl)amino}phenyl)diazene-1-yl]-1,3-thiazol-3-ium
- salts of 3-ethyl-2-[2-(4-{[4-({4-[2-(3-ethyl-1,3-thiazol-3-ium-2-yl)diazene-1-yl]phenyl}(methyl)amino)butyl](methyl)amino}phenyl)diazene-1-yl]-1,3-thiazol-3-ium
- salts of 3-ethyl-2-[2-(4-{[5-({4-[2-(3-ethyl-1,3-thiazol-3-ium-2-yl)diazene-1-yl]phenyl}(methyl)amino)pentyl](methyl)amino}phenyl)diazene-1-yl]-1,3-thiazol-3-ium
- salts of 3-ethyl-2-(2-{4-[ethyl({3-[ethyl({4-[2-(3-ethyl-1,3-thiazol-3-ium-2-yl)diazene-1-yl]phenyl})amino]propyl})amino]phenyl}diazene-1-yl)-1,3-thiazol-3
- salts of 3-ethyl-2-(2-(4-[ethyl({4-[ethyl({4-[2-(3-ethyl-1,3-thiazol-3-ium-2-yl)diazene-1-yl]phenyl})amino]butyl})amino]phenyl}diazene-1-yl)-1,3-thiazol-3-ium and/or
- salts of 3-ethyl-2-(2-{4-[ethyl({5-[ethyl({4-[2-(3-ethyl-1,3-thiazol-3-ium-2-yl)diazene-1-yl]phenyl})amino]pentyl})amino]phenyl}diazene-1-yl)-1,3-thiazol-3-ium.

3. The agent according to one of claims 1 to 2, **characterized in that** it contains, based on the total weight of the agent, one or more substantive dyes (a) of formula (I) in a total amount of from 0.01 to 4.5 wt.%, preferably from 0.05 to 2.8 wt.%, more preferably from 0.1 to 2.2 wt.% and particularly preferably from 0.2 to 1.2 wt.%.

4. The agent according to one of claims 1 to 3, **characterized in that** it contains (b) at least one anionic surfactant which is selected from
- linear and branched fatty acids having 8 to 30 C atoms,
- ether carboxylic acids of formula R-O-(CH₂-CH₂O)ₓCH₂-COOM, in which
R represents a linear alkyl group having 8 to 30 C atoms;
x represents an integer from 0 to 16;
M represents hydrogen or a metal such as alkali metal, in particular sodium, potassium, lithium, alkaline earth metal, in particular magnesium, calcium, zinc, or an ammonium ion (NH4⁺);
- acyl sarcosides having 8 to 24 C atoms in the acyl group;
- acyl taurides having 8 to 24 C atoms in the acyl group;
- acyl isethionates having 8 to 24 C atoms in the acyl group, which can be obtained by esterification of C₈-C₂₄ fatty acids with the sodium salt of 2-hydroxyethanesulfonic acid (isethionic acid);
- amide ether carboxylic acids, R¹-CO-NR²-CH₂CH₂-O-(CH₂CH₂O)_{y}CH₂COOM, where
R¹ represents a C2-C30 alkyl group,
y represents an integer from 1 to 20,
R² represents hydrogen, a methyl, ethyl, propyl, isopropyl, n-butyl, t-butyl or iso-butyl functional group, and
M represents hydrogen or a metal such as alkali metal, in particular sodium, potassium, lithium, alkaline earth metal, in particular magnesium, calcium, zinc, or an ammonium ion (NH4⁺).

5. The agent according to one of claims 1 to 4, **characterized in that** it contains, as the anionic surfactant (b), at least one ether carboxylic acid of formula (B1),
R-O-(CH2-CH2O)x-CH2-COOM (B1),
where
R represents a linear alkyl group having 8 to 30 C atoms;
x represents an integer from 0 to 16;
M represents hydrogen or a metal such as an alkali metal, in particular sodium, potassium, lithium, an alkaline earth metal, in particular ½ magnesium, ½ calcium, ½ zinc, or an ammonium ion (NH4⁺),

6. The agent according to one of claims 1 to 5, **characterized in that** it contains, based on the total weight of the agent, one or more anionic surfactants (b) in a total amount of from 0.05 to 4.5 wt.%, preferably from 0.1 to 3.1 wt.%, more preferably from 0.15 to 2.5 wt.% and very particularly preferably from 0.2 to 0.9 wt.%.

7. The agent according to one of claims 1 to 6, **characterized in that** the weight ratio of all the anionic surfactants of formula (B1) contained in the agent with respect to the total amount of the anionic surfactants contained in the agent is 0.5, preferably 0.6, more preferably 0.75 and particularly preferably 0.9.

8. The agent according to one of claims 1 to 7, **characterized in that** it additionally contains at least one further cationic substantive dye that is different from the dyes of formula (I).

9. The agent according to one of claims 1 to 8, **characterized in that** it additionally contains Basic Orange 31 and/or Basic Red 51.

10. The agent according to one of claims 1 to 9, **characterized in that** it contains, based on the total weight of the agent, 0.5 to 12.5 wt.%, preferably 2.5 to 10 wt.%, and in particular 3 to 6 wt.% of hydrogen peroxide.

11. The agent according to one of claims 1 to 10, **characterized in that** it additionally contains at least one persulfate from the group ammonium peroxodisulfate, potassium peroxodisulfate and sodium peroxodisulfate.

## Revendications

1. Agent de coloration de fibres kératiniques, en particulier de cheveux humains, contenant, dans un support cosmétique,
(a) au moins un colorant direct de formule (I), 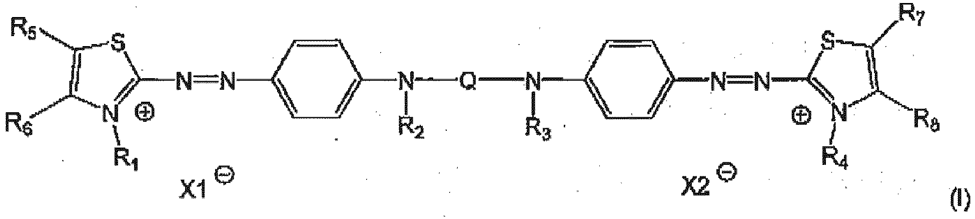 dans laquelle
R1 et R4 représentent indépendamment un groupe alkyle C₁-C₈,
R2 et R3 représentent indépendamment un atome d'hydrogène ou un groupe alkyle C₁-C₈,
R5, R6, R7 et R8 représentent chacun un atome d'hydrogène,
X1 et X2 représentent indépendamment un anion physiologiquement compatible, de préférence compris dans le groupe constitué par le chlorure, le bromure, l'iodure, le méthylsulfate, le méthylsulfonate, le p-toluènesulfonate, l'acétate, le sulfate d'hydrogène, le ½ sulfate ou le ½ tétrachlorozincate,
Q représente un groupe de formule (II), *-(CH₂)n-* (II)
n représente un nombre entier compris entre 3 et 6, et
(b) au moins un tensioactif anionique.

2. Agent selon la revendication 1, **caractérisé en ce qu'**il contient (a) au moins un colorant direct de formule générale (I), choisi parmi
- des sels du 3-méthyl-2-[2-(4-{[3-({4-[2-(3-méthyl-1,3-thiazol-3-ium-2-yl)diazène-1-yl]phényl}amino)propyl]amino}phényl)diazène-1-yl]-1,3-thiazol-3-ium
- des sels du 3-méthyl-2-[2-(4-{[4-({4-[2-(3-méthyl-1-3-thiazol-3-ium-2-yl)diazène-1-yl]phényl}amino)butyl]amino}phényl)diazène-1-yl]-1,3-thiazol-3-ium
- des sels du 3-méthyl-2-[2-(4-{[5-({4-[2-(3-méthyl-1,3-thiazol-3-ium-2-yl)diazène-1-yl]phényl}amino)pentyl]amino}phényl)diazène-1-yl]-1,3-thiazol-3-ium
- des sels du 3-méthyl-2-(2-(4-[méthyl({3-[méthyl({4-[2-(3-méthyl-1,3-thiazol-3-ium-2-yl)diazène-1-yl]phényl})amino]propyl})amino]phényl}diazène-1-yl)-1,3-thiazol-3-ium
- des sels du 3-méthyl-2-(2-{4-[méthyl({4-[méthyl({4-[2-(3-méthyl-1,3-thiazol-3-ium-2-yl)diazène-1-yl]phényl})amino]butyl})amino]phényl}diazène-1-yl)-1,3-thiazol-3-ium
- des sels du 3-méthyl-2-(2-{4-[méthyl({5-[méthyl({4-[2-(3-méthyl-1,3-thiazol-3-ium-2-yl)diazène-1-yl]phényle})amino]pentyl})amino]phényl)diazène-1-yl)-1,3-thiazol-3-ium
- des sels du 2-(2-{4-[éthyl({3-[éthyl({4-[2-(3-méthyl-1,3-thiazol-3-ium-2-yl)diazène-1-yl]phényl})amino]propyl})amino]phényl}diazène-1-yl)-3-méthyl-1,3-thiazol-3-ium
- des sels du 2-(2-{4-[éthyl({4-[éthyl({4-[2-(3-méthyl-1,3-thiazol-3-ium-2-yl)diazène-1-yl]phényl})amino]butyl})amino]phényl}diazène-1-yl)-3-méthyl-1,3-thiazol-3-ium
- des sels du 2-(2-{4-[éthyl({5-[éthyl({4-[2-(3-méthyl-1,3-thiazol-3-ium-2-yl)diazène-1-yl]phényl})amino]pentyl})amino]phényl}diazène-1-yl)-3-méthyl-1,3-thiazol-3-ium
- des sels du 3-éthyl-2-[2-(4-{[3-({4-[2-(3-éthyl-1,3-thiazol-3-ium-2-yl)diazène-1-yl]phényl}amino)propyl]amino}phényl)diazenène-1-yl]-1,3-thiazol-3-ium
- des sels du 3-éthyl-2-[2-(4-{[4-({4-[2-(3-éthyl-1,3-thlazol-3-ium-2-yl)diazène-1-yl]phényl}amino)butyl]amino}phényl)diazène-1-yl]-1,3-thiazol-3-ium
- des sels du 3-éthyl-2-[2-(4-{[5-({4-[2-(3-éthyl-1,3-thiazol-3-ium-2-yl)diazène-1-yl]phényl}amino)pentyl]amino}phényl)diazène-1-yl]-1,3-thiazol-3-ium
- des sels du 3-éthyl-2-[2-(4-{[3-({4-[2-(3-éthyl-1,3-thiazol-3-ium-2-yl)diazène-1-yl]phényl}(méthyl)amino)propyl](méthyl)amino}phényl)diazène-1-yl]-1,3-thiazol-3-ium
- des sels du 3-éthyl-2-[2-(4-{[4-({4-[2-(3-éthyl-1,3-thiazol-3-ium-2-yl)diazène-1-yl]phényl}(methyl)amino)butyl](méthyl)amino}phényl)diazène-1-yl]-1,3-thiazol-3-ium
- des sels du 3-éthyl-2-[2-(4-{[5-({4-[2-(3-éthyl-1,3-thiazol-3-ium-2-yl)diazène-1-yl]phényl}(méthyl)amino)pentyl](méthyl)amino}phényl)diazène-1-yl]-1,3-thiazol-3-ium
- des sels du 3-éthyl-2-(2-{4-[éthyl({3-[éthyl({4-[2-(3-éthyl-1,3-thiazol-3-ium-2-yl)diazène-1-yl]phényl})amino]propyl})amino]phényl}diazène-1-yl)-1,3-thiazol-3
- des sels du 3-éthyl-2-(2-(4-[éthyl({4-[éthyl({4-[2-(3-éthyl-1,3-thiazol-3-ium-2-yl)diazène-1-yl]phényl})amino]butyl})amino]phényl}diazène-1-yl)-1,3-thiazol-3-ium et/ou
- des sels du 3-éthyl-2-(2-{4-[éthyl({5-[éthyl({4-[2-(3-éthyl-1,3-thiazol-3-ium-2-yl)diazène-1-yl]phényl})amino]pentyl})amino]phényl}diazène-1-yl)-1,3-thiazol-3-ium.

3. Agent selon l'une des revendications 1 à 2, **caractérisé en ce qu'**il contient, par rapport au poids total de l'agent, au moins un colorant direct (a) de formule (I) en une quantité totale comprise entre 0,01 et 4,5 % en poids, de préférence entre 0,05 et 2,8 % en poids, de manière davantage préférée entre 0,1 et 2,2 % en poids et de manière particulièrement préférée entre 0,2 et 1,2 % en poids.

4. Agent selon l'une des revendications 1 à 3, **caractérisé en ce qu'**il contient (b) au moins un tensioactif anionique, choisi parmi
- les acides gras linéaires et ramifiés ayant 8 à 30 atomes de carbone,
- les acides éther carboxyliques de formule R-O-(CH₂-CH₂O)ₓ-CH₂-COOM, dans laquelle
R représente un groupe alkyle linéaire ayant 8 à 30 atomes de carbone,
x représente un nombre entier compris entre 0 et 16,
M représente un atome d'hydrogène ou un métal tel qu'un métal alcalin, en particulier le sodium, le potassium, le lithium, un métal alcalino-terreux, en particulier le magnésium, le calcium, le zinc ou un ion ammonium (NH4⁺),
- les acylsarcosides ayant 8 à 24 atomes de carbone dans le groupe acyle,
- les acyltaurates ayant 8 à 24 atomes de carbone dans le groupe acyle,
- les acyliséthionates ayant 8 à 24 atomes de carbone dans le groupe acyle, lesquels sont obtenus par estérification des acides gras C₈-C₂₄ avec du sel de sodium de l'acide 2-hydroxyéthane-sulfonique (acide iséthionique),
- Les acides amidoéthercarboxyliques de formule R¹-CO-NR²-CH₂CH₂-O-(CH₂CH₂O)_{y}CH₂COOM, dans laquelle
R¹ représente un groupe alkyle C2-C30,
y représente un nombre entier compris entre 1 et 20,
R² représente un atome d'hydrogène, un radical méthyle, éthyle, propyle, isopropyle, n-butyle, t-butyle ou isobutyle, et
M représente un atome d'hydrogène ou un métal tel qu'un métal alcalin, en particulier le sodium, le potassium, le lithium, un métal alcalino-terreux, en particulier le magnésium, le calcium, le zinc ou un ion ammonium (NH4⁺).

5. Agent selon l'une des revendications 1 à 4, **caractérisé en ce qu'**il contient, comme tensioactif anionique (b), au moins un acide éther-carboxylique de formule (B1),
R-O-(CH2-CH2O)x-CH2-COOM (B1),
dans laquelle
R représente un groupe alkyle linéaire ayant 8 à 30 atomes de carbone,
x représente un nombre entier compris entre 0 et 16,
M représente un atome d'hydrogène ou un métal tel qu'un métal alcalin, en particulier le sodium, le potassium, le lithium, un métal alcalino-terreux, en particulier le ½ magnésium, le ½ calcium, le ½ zinc ou un ion ammonium (NH4⁺).

6. Agent selon l'une des revendications 1 à 5, **caractérisé en ce qu'**il contient, par rapport au poids total de l'agent, au moins un colorant direct (b) en une quantité totale comprise entre 0,05 et 4,5 % en poids, de préférence entre 0,1 et 3,1 % en poids, de manière davantage préférée entre 0,15 et 2,5 % en poids et de manière particulièrement préférée entre 0,2 et 0,9 % en poids.

7. Agent selon l'une des revendications 1 à 6, **caractérisé en ce que** le rapport en poids de tous les agents tensioactifs anioniques de formule (B1) présents dans l'agent à la quantité totale des agents tensioactifs anioniques présents dans l'agent se situe à une valeur de 0,5, de préférence de 0,6, de manière davantage préférée de 0,75 et de manière particulièrement préférée de 0,9.

8. Agent selon l'une des revendications 1 à 7, **caractérisé en ce qu'**il contient en outre au moins un autre colorant direct cationique qui est différent des colorants de formule (I).

9. Agent selon l'une des revendications 1 à 8, **caractérisé en ce qu'**il contient en outre Basic Orange 31 et/ou Basic Red 51.

10. Agent selon l'une des revendications 1 à 9, **caractérisé en ce qu'**il contient, par rapport au poids total de l'agent, 0,5 à 12,5 % en poids, de préférence 2,5 à 10 % en poids et en particulier 3 à 6 % en poids de peroxyde d'hydrogène.

11. Agent selon l'une des revendications 1 à 10, **caractérisé en ce qu'**il contient en outre au moins un persulfate choisi dans le groupe constitué par le peroxodrsulfate d'ammonium, le peroxodisulfate de potassium et le peroxodisulfate de sodium.
